# EUROPEAN PATENT SPECIFICATION

(11) **EP 2 286 666 B1**
(45) Date of publication and mention of the grant of the patent: **26.12.2012**
(21) Application number: 08752270.2
(22) Date of filing: 01.05.2008
(51) Int. Cl.: A01N 25/30, A01N 59/16, A01N 37/06, A01N 37/10, A01N 37/44, A01N 41/04, A01N 43/08, A01N 59/20, A01P 3/00, A61K 31/198, A61K 9/08, A61K 31/375, A61K 33/24, A61K 33/26, A61K 33/30, A61K 33/34, A61K 33/38, A61K 45/06, A61K 47/12, A23B 4/18, A23B 5/12

(54) **VERSATILE DISINFECTANT**
VIELSEITIGES DESINFEKTIONSMITTEL
DÉSINFECTANT POLYVALENT

(43) Date of publication of application: 23.02.2011
(73) Proprietor: Anthrax Spores Killer Co., Limited, Hong Kong (CN)
(72) Inventor: HATA, Yadayo, Osaka-shi Osaka 545-0053 (JP); HATA, Tomoyo, Osaka-shi Osaka 545-0053 (JP); TOSHIMORI, Hitoshi, Osaka-shi Osaka 543-0074 (JP); MIYAZAWA, Masaaki, Osakasayama-shi Osaka 589-0023 (JP); OTSUKI, Koichi, Kyoto-shi Kyoto 606-0022 (JP); TAKAKUWA, Hiroki, Kyoto-shi Kyoto 606-0014 (JP); MARUOKA, Toshiyuki, Toyonaka-shi Osaka 560-0033 (JP)
(74) Representative: von Uexküll - Güldenband, Alexa
(86) International application number: PCT/JP2008/058340
(87) International publication number: WO 2009/133616

(56) References cited:
- JP-A- 6 306 082
- JP-A- 7 316 009
- JP-A- 2000 044 417
- JP-A- 2000 226 398
- US-B1- 6 296 881
- S.Y. LIAU ET AL: "Interaction of silver nitrate with readily identifiable groups: relationship to the antibacterialaction of silver ions", LETTERS IN APPLIED MICROBIOLOGY, vol. 25, no. 4, 1 October 1997 (1997-10-01), pages 279-283, XP55004345, ISSN: 0266-8254, DOI: 10.1046/j.1472-765X.1997.00219.x
- MATSUMURA Y ET AL: "Mode of bactericidal action of silver zeolite and its comparison with that of silver nitrate", APPLIED AND ENVIRONMENTAL MICROBIOLOGY, AMERICAN SOCIETY FOR MICROBIOLOGY, US, vol. 69, no. 7, 1 July 2003 (2003-07-01), pages 4278-4281, XP002308887, ISSN: 0099-2240, DOI: 10.1128/AEM.69.7.4278-4281.2003
- TOTSUKA A ET AL: "THE EFFECTS OF AMINO ACIDS AND METALS ON THE INFECTIVITY OF POLIOVIRUS RIBONUCLEIC ACID", JAPANESE JOURNAL OF MICROBIOLOGY, TOKYO, JP, vol. 18, no. 2, 1 March 1974 (1974-03-01), pages 107-112, XP000644692, ISSN: 0021-5139
- WEINBERG E D: "The role of cobalt in amino acid antagonisms", ANTONIE VAN LEEUWENHOEK, vol. 26, 1 December 1960 (1960-12-01), pages 321-328, XP009151154, DOI: 10.1007/BF02539020
- KONOWALCHUK J, HINTON N A, REED G B: "ANTIBACTERIAL ACTION OF A REACTION PRODUCT OF CYSTEINE AND IRON: I. DEVELOPMENT OF THE SUBSTANCE IN MEDIA FOR MYCOBACTERIUM TUBERCULOSIS", CANADIAN JOURNAL OF MICROBIOLOGY, vol. 1, 1 December 1954 (1954-12-01), pages 175-181, XP009151155, DOI: 10.1139/m55-023
- ENRIQUEZ-OBREGON G A ET AL: "AGROBACTERIUM-MEDIATED JAPONICA RICE TRANSFORMATION: A PROCEDURE ASSISTED BY AN ANTINECROTIC TREATMENT", PLANT CELL, TISSUE AND ORGAN CULTURE, SPRINGER, NL, vol. 59, no. 3, 1 January 1999 (1999-01-01), pages 159-168, XP000926499, ISSN: 0167-6857, DOI: 10.1023/A:1006307527449

## Description

### Technical Field

The present invention relates to a novel disinfectant that includes as main components an amino acid, a vitamin, and a mineral that are basic substances of life and vital activities, that has extremely high safety and a broad antimicrobial spectrum, that can be used for various purposes and applications, for example, the disinfections of fingers, mucosae, wounds, instruments, equipments, excrements, environments, as well as fresh foods, and crops, and that does not follow the conventional concepts.

### Background Art

The history of human beings in battle against pathogens has started at the moment when the human beings were generated, and the human beings had been defeated one-sidedly by unapparent foes without any effective weapons for a long time and had only been frightened.

Then, the change from the hunting society to the agrarian society has promoted the settlement of human beings, and increase population density has caused the outbreak of infection by droplets and contact. When the transportation means such as Silk Road, great voyages, Trans-African Highway have been developed, characteristic infections such as pest, cholera, syphilis, and malaria have been spread. For example, smallpox has emerged in ancient India and China, then spread out to Middle East and Egypt, broken into Europe with Crusade, and then reached the American Continent with the discovery of the New Continent. The syphilis has come into Europe from America through the reverse route.

The discovery of Bacillus anthracis by Koch in 1876 has revealed that microorganisms cause infections by which the human beings have been affected. After that, infections and causative pathogens have been gradually identified, and then the true battle of human beings against the pathogens has been started.

Human wisdom has created methods for attacking the pathogens. The development of effective disinfectants, antibiotics, and vaccines has likely brought the pathogens to bay to completely control them for a while. However, it has been realized that it was only a dream for a moment. Because the bacteria and viruses have been endured large change in environment to fit it and widened the area of activities since microorganisms emerged on the earth three billion years ago, they could not be easily defeated.

Overuse of antibiotics has generated methicillin-resistant Staphylococcus aureus (MRSA), vancomycin-resistant enterococci (VRE), multidrug-resistant Pseudomonas aeruginosa (MDRP), and multidrug-resistant tuberculosis (XMR-TB) that have no response on drugs. Economic overdevelopment has exposed Ebola haemorrhagic fever, West Nile fever, Lassa fever, spotted fever, Nipah virus encephalitis, and the like that were endemic in wild animals. In the future, unknown infections (emerging infections) of which the infection pathway and cause will not identified should continuously emerge crossing species barriers. In the recent case of highly pathogenic avian influenza, when a slight mutation in the virus actually causes infection from person to person (emerging of a new type of influenza), it may be estimated that the new type of influenza will spread rapidly all over the world (pandemic) to cause at least one hundred million deaths.

When analyzing such events, it seems that the pathogens have been changed in the way of attack against human beings in response to the social transformation such as the changes in our lifestyle and environment, the development of medical care, globalization, and aging. Human beings may be brittle with respect to the pathogens that have been mutated at a several ten millions times higher speed than that of the human beings to stay alive.

Examples of the means for protecting ourselves against such directly invisible fearful pathogens include the global improvement of public health system, the establishment of risk management, system, the rapid provision of information, and the complete sterilization and disinfection of pathogens with which someone may disagree.

Examples of conventional general-purpose disinfectants include alcohols, phenols, halogenated compounds, quaternary ammonium salts, biguanide drugs, and acetaldehydes. Recently, strongly acidic water, ozone water, and the like have been used for sterilizing fresh foods. Such disinfectants are desired to have the following features:
(1) a broad antimicrobial spectrum;
(2) capable of sterilization by short time contact (immediate efficacy);
(3) high stability and continuous bactericidal activity;
(4) the effect is less under the presence of organic substances;
(5) less harmful with respect to a living body (low toxicity);
(6) readily dissolved in water and easy to be used;
(7) high osmosis;
(8) no unpleasant odor;
(9) low cost;
(10) good preservability;
(11) readily discarded
(12) no damage on an object to be treated; and
(13) not to generate resistant bacteria.

However, as apparent from the relation between bacteria and antibiotics, the microorganisms will acquire the resistance sooner or later with respect to a pharmaceutical drug (compound) that attacks the microorganisms. This is an unavoidable fact, and there are a large number of reports on hospital infections that are not affected by alcohols, quaternary ammonium salts, or biguanide drugs.

Furthermore, no disinfectant satisfies all of the features (1) to (13) unfortunately. Therefore, conventional disinfectants have been selected depending on the most important feature among them for a purpose of use.

People have received benefit from modern medicine that had started in the mid-19th century. On the other hand, people have accepted some adverse effects and toxicity, and thus medicine has been developed and truth has been sought at the cost of people.
In the 21st century, "safety and security" become most important and most urgent in the society and public.

In view of the above problems in such state, the inventors have developed a disinfectant that almost satisfies the requirements, filed a patent for "Disinfectant Containing Iron Ion" in 1998, and have patented (Patent Document 1).
The disinfectant includes several compounds that are approved as food additives, and has low toxicity but inhibits the emergence of resistant bacteria in the same way as that of combination use of a plurality of pharmaceutical drugs for killing tubercle bacillus or Helicobacter pylori. In addition to the features, it has excellent features such as a broad antimicrobial spectrum and the effect on spores. However, the inventors
further studied on the disinfectant containing iron ions, and reveal have that the disinfectant is not perfect for users because the disinfectant itself and a treated object gradually discolor by the effect of Fe³⁺ ion as a main component (when left standing), the disinfectant has unpleasant odors and tastes, and when the disinfectant is dispersed over crops for disinfection, the growth of some crops is inhibited. Furthermore, the disinfectant containing iron ion has excellent sterilizing power with respect to various pathogens but has unsatisfied killing power with respect to viruses.
Patent Document 1: Japanese Patent No. 3853985

### Summary of Invention

### Technical Problem

In view of the above problems, it is an object of the present invention to provide a disinfectant that is almost non-toxic and harmless to human beings, animals, and plants and that has high sterilizing power against a wide range of pathogenic microorganisms and excellent continuous bactericidal activity, and to provide a sterilization method using the disinfectant by bringing it into contact with an environment, an instrument, a plant body or an organic substance.

Further more the the present invention provides for the use of the disinfectant in the adjunctive treatment of infections and for the use in the sterilization or disinfection of the skin of mucosa.

### Solution to Problem

The inventors of the present invention have carried out intensive studies in order to develop a disinfectant that follows the excellent sterilizing power and difficulty of resistance acquisition in the disinfectant according to Patent Document 1 previously developed by the inventors, but that solves the problems and has low toxicity and no side effect. First, the specification of Patent Document 1 has been verified in detail, and the reexamination has been carried out from other viewpoints. As a result, it is found that a disturbance phenomenon singular to microorganisms appears when a certain substance has a concentration that is the boundary between "plus" effect and "minus" effect on the growth of microorganisms, and that the phenomenon is captured to induce the growth to the "minus" side, thus bacteriostasis is expected, and the bacteriostasis is amplified to lead to the sterilization.

Based on the knowledge, the inventors have successfully developed an ideal versatile disinfectant that does not follow the conventional concepts, that includes, as main components, an amino acid, a vitamin, and a mineral that are basic substances of life and have high affinity to living bodies, and further includes a trace amount of a surfactant and the like with safety that is established from long-term use as daily necessities, and that is almost non-toxic to human beings, animals, and plants but has high sterilizing power against a wide range of pathogenic microorganisms by the synergistic effect and can be used for almost all objects.

That is, the present invention relates to:
[1] a disinfectant that includes one or more metal ions having antimicrobial activity, L-cysteine, and L-ascorbic acid as main components, and in addition to the main components, one or more surfactants other than a non-ionic surfactant;
[2] the disinfectant according to the item [1], in which the metal ion having antimicrobial activity is a trivalent iron ion (Fe³⁺), a divalent iron ion (Fe²⁺), a zinc ion (Zn²⁺), a copper ion (Cu²⁺), a cobalt ion (Co²⁺), a nickel ion (Ni²⁺), or a silver ion (Ag⁺);
[3] the disinfectant according to the item [2], in which the metal ion having antimicrobial activity has a concentration of 50 to 200 ppm in the case of the trivalent iron ion, 110 to 400 ppm in the case of the divalent iron ion, 7.5 to 125 ppm in the case of the zinc ion, 15 to 60 ppm in the case of the copper ion, 180 to 300 ppm in the case of the cobalt ion, 85 to 175 ppm in the case of the nickel ion, and 1 to 3 ppm in the case of the silver ion;
[4] the disinfectant according to any one of the items [1] to [3], in which the L-cysteine has a concentration of 100 to 1000 ppm and the L-ascorbic acid has a concentration of 100 to 500 ppm;
[5] the disinfectant according to any one of the items [1] to [4], in which the surfactant other than a non-ionic surfactant is one or more surfactants selected from the group consisting of an alkylbenzene sulfonate, a linear alkylbenzene sulfonate, a polyoxyethylene alkyl ether sulfate, a higher alcohol sulfate, sodium lauryl sulfate, sodium lauroyl sarcosinate, stearyl dimethyl benzyl ammonium chloride, benzalkonium chloride, benzethonium chloride, alkyldiaminoethylglycine hydrochloride, and alkylpolyaminoethylglycine hydrochloride;
[6] the disinfectant according to any one of the items [1] to [5], in which the surfactant other than a non-ionic surfactant has a concentration of 20 to 100 ppm;
[7] the disinfectant according to any one of the items [1] to [6], that further includes one or more compounds selected from the group consisting of sorbic acid, a sorbate, benzoic acid, a benzoate, and a para-oxybenzoate;
[8] the disinfectant according to the item [7], in which each of the sorbic acid, sorbate, benzoic acid, benzoate, and para-oxybenzoate has a concentration of 50 to 100 ppm;
[9] the disinfectant according to any one of the items [1] to [8], in which the disinfectant is adjusted to pH 2.5 to 4.0;
[10] a sterilization method that includes bringing the disinfectant according to any one of the items [1] to [9] into contact with an environment, an instrument, a plant body, or an organic substance.
[11] The disinfectant according to any of items 1 to 9 for use in the adjunctive treatment of infections in human beings and animals.
[12] The disinfectant according to any one of items 1 to 9 for use in the disinfection or sterilization of the skin or mucosa of a human or animal body.

### Advantageous Effects of Invention

The disinfectant of the present invention has extremely high safety because it includes as main components amino acids, vitamins, and minerals that are the bases of life and have high affinity to living bodies. In addition, introduction of a nonconventional concept into the disinfectant activates trace amount action of minerals to the maximum extent, and thus the disinfectant can sterilize and disinfect various pathogens including common bacteria, acid fast bacteria, spores, fungi, and viruses.

Furthermore, the disinfectant of the present invention can be effectively used directly for any object such as skins and mucosae as well as excrements, instruments, equipments, and environments. It can be also widely used indirectly for quasi-drugs such as therapeutic dentifrice, medicinal soap, and mouthwash, cosmetics such as shampoo and rinse, and pets for health and deodorant.

Furthermore, the disinfectant of the present invention has high utility value for cleaning fresh foods such as vegetables and fishes and for an adjunctive treatment of infections in human beings and animals.

### Brief Description of Drawing

Fig. 1 is a schematic view showing that a concentration sufficient for sterilization is in the range from the concentration at which bacteria antiproliferative activity is clearly observed to the concentration at which bacteriostasis is clearly observed.
Fig. 2 is a schematic explanatory view showing a change in the strength of antimicrobial activity when a metal ion and a food preservative are used in combination.

### Description of Embodiments

[I] In order to develop the disinfectant of the present invention, each component used in Patent Document 1 was reexamined.

### (1) Reexamination of metal ion (verification and examination of mineral component)

The minerals are one of the five major nutrients, provide various physiological actions in a living body, become the core of an enzyme that works for life source, and work as a catalyst.

Meanwhile, the antimicrobial activity of silver and copper has been empirically known from long ago and has applied in daily life, for example, to tableware, coins, and accessories. However, since the development of organic disinfectants, the metal is rarely used as an antimicrobial agent except in some products. In such circumstance, the inventors have used a trivalent iron ion (Fe³⁺) as a main component in the disinfectant according to
Patent Document 1 because the ion has comparatively low toxicity, is used as a food additive, and also has antimicrobial activity.

Such inorganic antimicrobial agents have low effect for a short time but basically have excellent advantages such as a broad antimicrobial spectrum and good stability and preservability. The reexamination was carried out on not only the Fe³⁺ ion but also essential minerals that are contained in tissues in living bodies and show antimicrobial activity in trace amounts (trace amount action of metals). As a result, a zinc ion (Zn²⁺), a copper ion (Cu²⁺), a cobalt ion (Co²⁺), a nickel ion (Ni²⁺), a divalent iron ion (Fe²⁺), and a silver ion (Ag⁺) that has been proven were selected, and the correlations between each ion concentration and the antimicrobial activities, that is, three steps of a) bacteria antiproliferative activity, b) bacteriostasis, and c) bactericidal activity were examined.
In the data described later in the present specification, "mineral ion" will be uniformly described as "metal ion" that is a common technical term.

### 1) When each metal ion is added to a growth medium

To a standard medium for counting viable bacteria described below, each metal ion with varied concentration was added. Next, a suspension of sample bacteria (1 × 10⁹ cells/ml physiological saline) was inoculated at 1% by weight. Then, the number of viable bacteria was counted with time in a common procedure. The correlation between each metal ion and the antimicrobial activity was observed from the viability to be listed in Table 1.
*Sample bacteria: S. aureus 209P as a representative of Gram-positive bacteria : E. coli 0-157 as a representative of Gram-negative bacteria
*standard medium for counting viable bacteria: yeast extract 2.5 g, peptone 5 g, glucose 1 g, agar 15 g, pH 7.2

[Table 1]

**Table 1-1 Antimicrobial Activity of Metal Ion**

| Metal ion (compound) | Antimicrobial activity | | | Acceleration of growth |
|---|---|---|---|---|
| | (a) Antiproliferative activity | (b) Bacteriostatic activity | (c) Bactericidal activity | |
| Fe⁺⁺ (FeCl₃ · 6H₂O) | 20~130 ppm | 120~500 ppm | 500 ppm> | 15 ppm< |
| Fe²⁺ (FeCl₂) | 35~180 ppm | 150~1000 ppm | 1000 ppm> | 30 ppm< |
| Zn²⁺ (ZnSO₄·7H₂O) | 12~18 ppm | 16~200 ppm | 200 ppm> | 10ppm< |
| Cu²⁺ <CuSO₄·5H₂O) | 10~50 ppm | 40-150 ppm | 150 ppm> | 5 ppm< |
| Co²⁺ (CoCl₂·6H₂O) | 100~250 ppm | 220~500 ppm | 500 ppm> | 20 ppm< |
| Ni²⁺ (NiSO₄·7H₂O) | 25~150 ppm | 120~300 ppm | 300 ppm> | 5 ppm< |

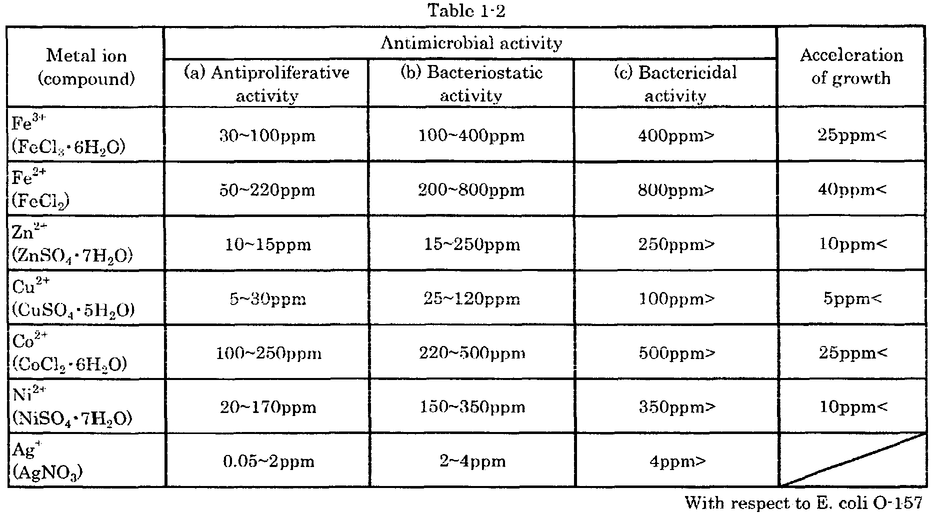

It is revealed that, when a metal ion is added to the medium and bacteria are cultured, the boundary of the concentration of metal ion is not clear between the antiproliferative activity and the bacteriostasis, but the bacteriostasis can be almost clearly distinguished from the bactericidal activity.
For example, in the case of Cu²⁺, the antiproliferative activity was observed from 10 to 50 ppm, the bacteriostasis was observed from 40 to 150 ppm, and the bactericidal activity was observed at 150 ppm or more.
Table 1 shows only the results obtained from typical compounds of each metal ion. However, other compounds listed below that can be dissolved in water to generate ions showed similar results: for example, as the Fe³⁺ ion, ferric chloride, ferric nitrate hexahydrate, ferric nitrate nonahydrate, ferric nitrate n-hydrate, ferric phosphate n-hydrate, and ferric citrate n-hydrate; as Fe²⁺ ion, ferrous chloride tetrahydrate, ferrous gluconate, ferrous citrate, and ferrous oxalate; as the Zn²⁺, zinc citrate dihydrate and zinc gluconate; as Cu²⁺, copper chloride dihydrate, copper(II) diammonium chloride dihydrate, and copper nitrate trihydrate; as Co²⁺, cobalt gluconate trihydrate, cobalt hydroxide, and cobalt citrate; as Ni²⁺, nickel nitrate; and as Ag⁺, silver sulfate and silver phosphate.

Here, the result shows that a metal ion (mineral) accelerates the growth at a suitable concentration.

(2) Based on the results in Tables 1-1 and 1-2, next, to an aqueous solution (purified water, at 28°C) dissolving each metal ion, a suspension of sample bacteria (1 × 10⁹ cells/1 ml water) was added at 1% by weight for contact. Then, each 0.2 ml of the solution was collected with time. The number of viable bacteria was immediately counted in a common procedure to calculate the death rate, and thus the correlation between the metal ion concentration and the antimicrobial activity was estimated. Staphylococcus aureus 209P and Escherichia coli 0-157 were used as the sample bacteria, but because both strain showed similar results, the mean values are shown in Table 2.

[Table 2]

**Table 2 Antimicrobial Activity of Metal Ion in Purified Water**

| Metal ion | Concentration ppm | Death rate over time | | | | | | | | |
|---|---|---|---|---|---|---|---|---|---|---|
| | | 1 | 3 | 5 | 10 | 15 | 30 | 60 | 90 | 120 min |
| Fe³⁺ | 23 | 0 | 0 | 0 | 5 | 10 | 20 | 25 | 40 | 70% |
| | 50 | 0 | 0 | 0 | 10 | 15 | 25 | 40 | 50 | 80% |
| | 200 | 0 | 0 | 5 | 15 | 25 | 30 | 50 | 65 | 90% |
| | 500 | 0 | 0 | 15 | 25 | 40 | 50 | 90 | 100% | |
| Fe²⁺ | 25 | 0 | 0 | 0 | 0 | 10 | 15 | 20 | 30 | 60% |
| | 100 | 0 | 0 | 0 | 10 | 20 | 30 | 40 | 45 | 70% |
| | 500 | 0 | 0 | 5 | 15 | 25 | 35 | 50 | 65 | 80% |
| | 1000 | 0 | 0 | 10 | 20 | 35 | 50 | 70 | 85 | 100% |
| Zn²⁺ | 5 | 0 | 0 | 0 | 5 | 10 | 15 | 25 | 50 | 80% |
| | 12 | 0 | 0 | 5 | 10 | 20 | 25 | 40 | 65 | 90% |
| | 100 | 0 | 0 | 5 | 20 | 30 | 40 | 60 | 80 | 100% |
| | 500 | 0 | 0 | 10 | 25 | 50 | 65 | 100% | | |
| Cu²⁺ | 2 | 0 | 0 | 5 | 10 | 20 | 35 | 50 | 60 | 85% |
| | 20 | 0 | 0 | 10 | 20 | 35 | 50 | 70 | 85 | 100% |
| | 50 | 0 | 0 | 10 | 30 | 50 | 70 | 90 | 100% | |
| | 200 | 0 | 10 | 15 | 40 | 60 | 80 | 100% | | |
| Co²⁺ | 100 | 0 | 0 | 0 | 0 | 5 | 10 | 20 | 30 | 75% |
| | 200 | 0 | 0 | 0 | 5 | 10 | 15 | 25 | 45 | 85% |
| | 350 | 0 | 0 | 0 | 10 | 20 | 25 | 50 | 65 | 100% |
| | 500 | 0 | 0 | 0 | 15 | 20 | 50 | 70 | 100% | |
| Ni²⁺ | 10 | 0 | 0 | 0 | 5 | 10 | 15 | 20 | 30 | 60% |
| | 50 | 0 | 0 | 0 | 5 | 15 | 25 | 35 | 50 | 70% |
| | 15U | 0 | 0 | 5 | 10 | 25 | 35 | 50 | 70 | 90% |
| | 300 | 0 | 0 | 10 | 15 | 40 | 50 | 75 | 90 | 100% |
| Ag⁺ | 0.01 | 0 | 0 | 0 | 15 | 35 | 70 | 100% | | |
| | 0.1 | 0 | 0 | 0 | 30 | 50 | 85 | 100% | | |
| | 3 | 0 | 10 | 20 | 40 | 80 | 100% | | | |
| | 10 | 0 | 25 | 35 | 50 | 100 | | | | |
| Physiological saline | | 0 | 0 | 0 | 0 | 0 | 0 | 0.5 | 0.7 | 1% |
| Purified water | | 0 | 0 | 0 | 0 | 0 | 1 | 1.5 | 2 | 3% |

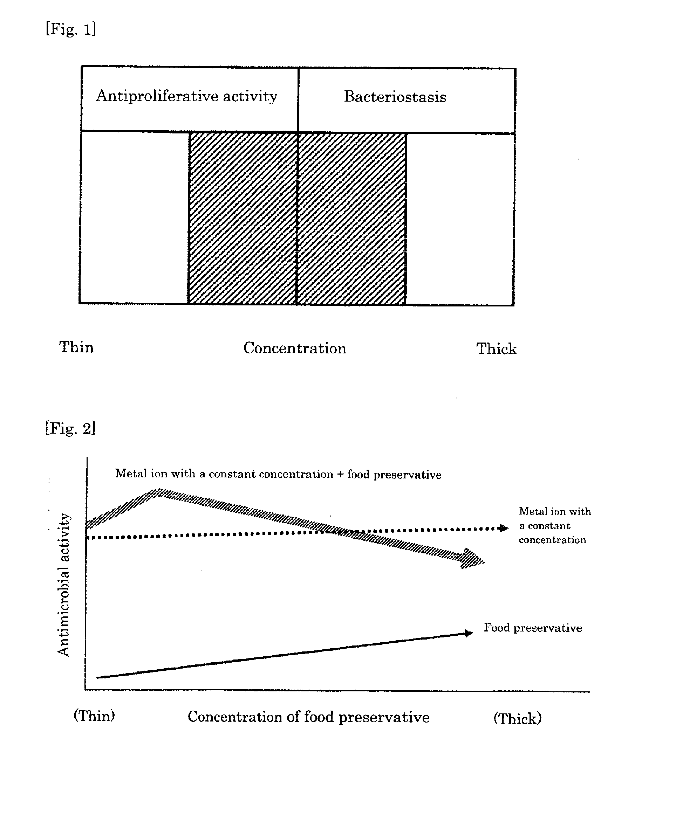

The results reveals the followings: even in the purified water without metal ions, the death rates were about 2% after 60 minutes and about 3% after 120 minutes (autolysis); in purified water with a metal ion, the ion concentration is proportional to the death rate, but when the contact time is as short as about 10 to 15 minutes, the death rate is not so affected by the concentrations and even when the concentration is extremely low but the contact time is long enough, there is not a sharp effect but the bacteria are killed with the speed gradually accelerated. From the test data concerning the metal ions in Table 1 and Table 2, it is assumed that:
<1> when a metal ion is added to a medium at a certain concentration and the proliferation of bacteria is inhibited, the route to bactericidal activity continues even at such concentration;
<2> when the sterilization is expected by a short time contact, a concentration sufficient for sterilization is in a range from the concentration at which bacteria antiproliferative activity is clearly observed to the concentration at which bacteriostasis is clearly observed (the diagonally shaded areas in Fig. 1);
<3> that is, even when a metal ion is brought into contact with bacteria at a concentration showing bactericidal activity, strong killing activity is not expected; and
<4> accordingly, with consideration of safety, the concentration is in a range shown in <2>, and insufficiency is preferably supplemented with other compounds to provide strong bactericidal activity to the maximum extent.

### (2) Verification and Reexamination of Food Preservative

In Patent Document 1, a food preservative is added in order to enhance and ensure the sterilization effect of the Fe³⁺ ion.

The inventors carefully examined the matters.

Food preservatives such as sorbic acid, sorbates, benzoic acid, benzoates, and para-oxybenzoates have poor bactericidal properties and work as bacteriostatic agents. They may inhibit the action of dehydrogenase in microorganisms and thus affect the growth adversely.

### 1) When various food preservatives are added to a growth medium

Following the above test on metal ions, a similar test was carried out and the results are summarized in Table 3 that shows the correlation of the concentrations of food preservatives with a) bacteria antiproliferative activity and with b) bacteriostasis.

[Table 3]

**Table 3 Antimicrobial Activitv of Food Preservative**

| Food preservative | Concentration of antimicrobial activity | | | |
|---|---|---|---|---|
| | (a) Antiproliferative activity | | (b) Bacteriostatic activity | |
| | S. aureus 209P | E. coli O-157 | S. aureus 209 | E. coli O-157 |
| Sorbic acid | 20~200 ppm | 20~200 ppm | 150 ppm> | 200 ppm> |
| Potassium sorbate | 35~250 ppm | 30~250 ppm | 200 ppm> | 250 ppm> |
| Benzoic acid | 25~200 ppm | 25~200 ppm | 180 ppm> | 180 ppm> |
| Sodium benzoate | 40~300 ppm | 40~300 ppm | 250 ppm> | 220 ppm> |
| Ethyl para-hydroxybenzoate | 50~300 ppm | 40~300 ppm | 250 ppm> | 230 ppm> |

As apparent from Table 3, the antimicrobial activity is not largely affected by the type of food preservatives. Each proliferation activity is observed at a concentration of 20 to 300 ppm, and each bacteriostasis is observed at a concentration of 200 to 300 ppm or more. The boundary between both activities is unclear and the active concentrations are overlapped.

### 2) When a food preservative is dissolved in purified water

Based on the results in Table 3, a similar test was carried out as that on the metal ions. The pH was adjusted to 3.0 because the effect is enhanced in an acidic region.

[Table 4]

**Table 4 Death Rate over Time by Food Preservative**

| | Concentration | Death rate over time | | | | | | |
|---|---|---|---|---|---|---|---|---|
| | | 5 | 10 | 15 | 30 | 60 | 90 | 120 min |
| Sorbic acid | 50 ppm | 0 | 0 | 0 | 2 | 5 | 10 | 15 |
| | 200 ppm | 0 | 0 | 0 | 2 | 7 | 12 | 18 |
| | 500 ppm | 0 | 0 | 1 | 3 | 10 | 15 | 20 |
| Potassium sorbate | 50 ppm | 0 | 0 | 0 | 2 | 5 | 8 | 12 |
| | 200 ppm | 0 | 0 | 0 | 2 | 6 | 10 | 15 |
| | 500 ppm | 0 | 0 | 1 | 3 | 7 | 12 | 18 |
| Benzoic acid | 50 ppm | 0 | 0 | 0 | 1 | 5 | 10 | 12 |
| | 200 ppm | 0 | 0 | 0 | 2 | 7 | 10 | 12 |
| | 500 ppm | 0 | 0 | 1 | 3 | 8 | 12 | 15 |
| Sodium benzoate | 50 ppm | 0 | 0 | 0 | 2 | 5 | 8 | 10 |
| | 200 ppm | 0 | 0 | 0 | 2 | 5 | 10 | 12 |
| | 500 ppm | 0 | 0 | 1 | 3 | 8 | 12 | 15 |
| Ethyl parahydroxybenzoate | 50 ppm | 0 | 0 | 0 | 2 | 5 | 8 | 12 |
| | 200 ppm | 0 | 0 | 0 | 2 | 5 | 10 | 12 |
| | 500 ppm | 0 | 0 | 0 | 2 | 7 | 12 | 15 |
| Purified water | | 0 | 0 | 0 | 1 | 1.5 | 2 | 3 |

When food preservatives were added into purified water, each death rate is higher than that of the purified water without food preservatives, but it may be caused by acceleration of the "autolysis". That is, in contrast to the metal ions, the food preservatives have no sterilization effect. Furthermore, the death rate was not largely affected by the concentration. That is, when the food preservative was added at 500 ppm that is ten times of 50 ppm, the difference was a little. Therefore, it is suggested that a preservative with a higher concentration is needless for food preservation.

Next, in a similar way to that in Patent Document 1, effect of the combination use of a metal ion and a food preservative was verified.
A large number of the combination tests were carried out using seven metal ions each having different concentrations and five food preservatives each having different concentrations. As a result, it is clear that, in the case that a food preservative is added to an aqueous metal ion having a concentration between the concentration at which antiproliferative activity is observed and the concentration at which bacteriostasis is observed, when the amount added is 200 ppm or more, metal ions other than the iron ions Fe³⁺ and Fe²⁺ have decreased sterilization effect, and a trace addition (50 to 100 ppm) eventually provides additive effect. (See Fig. 2)

### (3) Verification on L-Ascorbic Acid

Next, L-ascorbic acid will be discussed.

L'ascorbic acid (vitamin C) is essential for a living body and well fitted to body tissues. Addition of a small amount of L-ascorbic acid increases affinity to cellular tissues. The investigation by the inventors reveals that an aqueous L-ascorbic acid solution itself has almost no antimicrobial activity, but its strong anti-oxidation activity stabilizes a metal ion and a food preservative and keeps their activities, and the contact with organic substances such as bacteria amplifies the antimicrobial activity of metal ions to give strong adverse effect to pathogens.

However, it is also clear that, in the case that L-ascorbic acid is added a large amount, the antimicrobial effect is decreased conversely.

For example, in the case that the Fe³⁺ ion is used at 500 ppm or more, L-ascorbic acid is recommended to be used at a concentration of 500 to 2000 ppm. However, it is ascertained that, in order to sufficiently provide the trace amount action of metal ions of the present invention, the lower concentration, that is, 100 to 500 ppm is preferred, and that L-ascorbic acid is not always essential depending on the use of L-cysteine, surfactants, or the like that are described later and their amount added.

As described hereinbefore, main components in the invention according to Patent Document 1 were verified and reexamined, and as a result, a too high concentration of the Fe³⁺ ion as a core component must contribute the coloring, tastes, odors, and adverse effect on plants.
Furthermore, it is ascertained that addition of a certain amount or more of a food preservative as an enhancing component further accelerates the coloring. (Oxidation reaction)

Addition of a large amount of L-ascorbic acid that inhibits oxidation reaction and enhances sterilization should be avoided because the sterilizing power is reduced.
That is, it is shown that too much amount and concentration of the components including the Fe³⁺ ion causes the various problems described above.
Therefore, intensive studies have been carried out in order to solve the problems at once, and as a result, in the disinfectant of the present invention, not only Fe³⁺ but also each ion of Fe²⁺, Zn²⁺, Cu²⁺, Co²⁺, Ni²⁺, and Ag⁺ each having the trace amount action are employed, considering the safety with respect to animals, plants, and environments, the used amount is minimized to provide the activities to the maximum extent, and food preservatives are not basically used when a metal ion has a low concentration because the additive effect is small (when used, a trace amount).
In addition, a small amount of L-ascorbic acid is added because each amount added of the above components is small. The above matters are considered as the base, (A) in addition to the metal ion component having antimicrobial activity, a compound for enhancing the sterilizing power is identified, and (B) a method for rapidly impregnating such compound into pathogens is studied. That is, a combination of (A) and (B) may provide the trace amount action of metal ions having antimicrobial activity maximally.

As a result, some expected compounds and methods satisfying (A) and (B), respectively, are screened through detailed tests. Finally, as the combination of (A) and (B), surfactants other than a non-ionic surfactant (anionic surfactants, cationic surfactants, and ampholytic surfactants) and L-cysteine are used as (A), then the osmotic pressure is increased as (B), and consequently, the development of the disinfectant of the present invention that has safety, a broad antimicrobial spectrum, and highly convenience and is just the "versatile" has been successfully achieved.
Hereinafter, the disinfectant of the present invention will be described.

### [II] The Disinfectant of the Present Invention

The disinfectant of the present invention includes, as main components, a metal ion having antimicrobial activity, L-cysteine, and L-ascorbic acid, and in addition to the main components, a surfactant other than a non-ionic surfactant.

### (1) Metal Ion Having Antimicrobial Activity

The metal ion having antimicrobial activity may be various metal ions verified in the section [I], and examples of the metal ion include a trivalent iron ion (Fe³⁺), a divalent iron ion (Fe²⁺), a zinc ion (Zn²⁺), a copper ion (Cu²⁺), a cobalt ion (Co²⁺) a nickel ion (Ni²⁺), and a silver ion (Ag⁺). Such metal ions may be used alone or in combination.

The amount of the metal ion in the disinfectant of the present invention may be properly adjusted so as to provide desired sterilizing power. For example, the iron ion preferably has a concentration of 50 to 200 ppm, the divalent iron ion preferably has a concentration of 110 to 400 ppm, the zinc ion preferably has a concentration of 7.5 to 125 ppm, the copper ion preferably has a concentration of 15 to 60 ppm, the cobalt ion preferably has a concentration of 180 to 300 ppm, the nickel ion preferably has a concentration of 85 to 175 ppm, and the silver ion preferably has a concentration of 1 to 3 ppm.

Furthermore, as the metal ion, for example, the various compounds above described that are dissolved in water to become ions may be used. Examples for the Fe³+ ion include ferric chloride, ferric nitrate hexahydrate, ferric nitrate nonahydrate, ferric nitrate n-hydrate, ferric phosphate n-hydrate, and ferric citrate n-hydrate. Examples for the Fe²⁺ ion include ferrous chloride tetrahydrate, ferrous gluconate, ferrous citrate, and ferrous oxalate. Examples for the Zn²⁺ include zinc citrate dihydrate and zinc gluconate. Examples for the Cu²⁺ include copper chloride dihydrate, copper(II) diammonium chloride dihydrate, and copper nitrate trihydrate. Examples for the Co²⁺ include cobalt gluconate trihydrate, cobalt hydroxide, and cobalt citrate. Examples for the Ni²⁺ include nickel nitrate. Examples for the Ag⁺ include silver sulfate and silver phosphate.

### (2) L-Cysteine

L-cysteine is one of the sulfur-containing amino acids and the essential component for skin metabolism, helps collagen production, and inhibits melanin production in cooperation with L-ascorbic acid. It is a main component in skin, nails, and hairs and widely distributed in the body. L-cysteine itself unexpectedly provides antimicrobial activity depending on its using way. In addition, an SH group (a thiol group that is formed by binding of sulfur with hydrogen) in the molecular structure is bonded to a metal ion having antimicrobial activity to amplify the activity, and strong bactericidal properties are provided. Then, by DNA inhibition, enzyme deactivation, inhibition of metabolic functions, denaturation of proteins, and generation of free radicals, the decay of microorganisms is accelerated. Its strong anti-oxidation effect and reduction effect contribute to the stability of components. It has high affinity to a living body, is strongly attached to pathogens, and helps the osmosis. Its optimum concentration slightly varies depending on the type and concentration of a metal ion contained, but the concentration is preferably about several times higher than the ion concentration. For example, the content of L-cysteine is preferably 100 to 1000 ppm in the disinfectant of the present invention.

### (3) L-Ascorbic Acid

The action of L-ascorbic acid is as described above. The content of L-ascorbic acid is preferably 100 to 500 ppm in the disinfectant of the present invention.

### (4) Surfactants Other Than a Non-ionicSurfactant

The surfactant includes lipophilic groups that are readily bonded to oil and hydrophilic groups that are readily bonded to water as the basic structure. It has various actions such as wetting, moisture absorbing, impregnating, solubilizing, emulsifying, dispersing, foaming, lubricating, washing, antistatic, adsorbing, film forming, antimicrobial, cell membrane disturbance, and rust-inhibiting. It is mainly applied in our daily life, for example, to synthetic detergent, kitchen detergent, dentifrice, rinse, emulsifier, and fabric softener, and is now an essential compound.

Many of the surfactants have some of the actions. However, from the view point of the "sterilization and disinfection", examples of the surfactant other than a non-ionic surfactant that can provide excellent effect when used in the present invention include anionic surfactants, cationic surfactants, and ampholytic surfactants described below.

### (Anionic Surfactant)

Alkylbenzene sulfonates (ABS type), linear alkylbenzene sulfonates (LAS type), polyoxyethylene alkyl ether sulfates (AES type), sodium lauryl sulfate, sodium lauroyl sarcosinate, and higher alcohol sulfates (AS)

### (Cationic Surfactant)

Stearyl dimethyl benzyl ammonium chloride, benzalkonium chloride, and benzethonium chloride

### (Ampholytic Surfactant)

Alkyldiaminoethylglycine hydrochloride and alkylpolyaminoethylglycine hydrochloride

The surfactants other than a non-ionic surfactant may be used alone or in combination of two or more of them.

When the disinfectant of the present invention contains the surfactant other than a non-ionic surfactant at a concentration of 20 to 100 ppm, the bactericidal activity is enhanced and the sterilization time is remarkably reduced. Examples of the effect are shown below.

[Table 5]

**Table 5 Sterilization Effect by Adding Surfactants**

| Cu²⁺ ion | Surfactant (sodium lauryl sulfate) | L-ascorbic acid | Potassium sorbate | 100% death time | |
|---|---|---|---|---|---|
| | | | | S. aureus 209P | E. coli O-157 |
| 50 ppm | | | | 90 min | 90 min |
| 50 ppm | 50ppm | | | 1.5 min | 1 min |
| 50 ppm | | 100 ppm | | 60 min | 60 min |
| 50 ppm | | | 50 ppm | 75 min | 70 min |
| 50 ppm | 50ppm | 100 ppm | | 1 min | 1 min |
| 50 ppm | | 100 ppm | 50 ppm | 50 min | 45min |
| 50 ppm | 50ppm | 100 ppm | 50 ppm | 45 sec | 30 sec |

### (5) Others

When the disinfectant of the present invention contains one or more compounds selected from the group consisting of sorbic acid, sorbates, benzoic acid, benzoates, and para-oxybenzoates, the sterilizing power can be improved.

Examples of the sorbate include potassium sorbate and sodium sorbate. Examples of the benzoate include potassium benzoate, sodium benzoate, calcium benzoate, ammonium benzoate, and zinc benzoate.

Each of the sorbic acid, potassium sorbate, benzoic acid, sodium benzoate, and para-oxybenzoates preferably has a concentration of 50 to 100 ppm in the disinfectant.

The disinfectant of the present invention can be prepared by adding each of the various components into water and mixing them. The order of addition is not limited specifically. Furthermore, examples of the water used as a solvent include tap water, ion-exchanged water, pure water, and purified water, and they may be properly selected depending on the intended purpose.

### (6) pH

When the disinfectant of the present invention is adjusted to acidic, potency of each component in the disinfectant is kept and stabilized as well as the osmosis into pathogen is supported. The disinfectant of the present invention preferably has a pH of 2.5 to 4.0. Conventional pH adjusters may be used for adjusting pH.

### (7) Osmosis

Osmosis is a phenomenon that a solution having a low concentration is osmosed into a solution having a high concentration when the solutions having different concentrations are separated by a semipermeable membrane, and the strength is referred to as osmotic pressure. Each of the main components included in the disinfectant of the present invention has high affinity to cellular tissues. Therefore, when the amount of each component is made as small as possible (low concentration), osmotic force is applied to a solution having a higher concentration (microorganisms inside), and thus the disinfectant gains immediate efficacy.

There is a general knowledge that an agent having a higher concentration is more effective, and it is not limited to disinfectants. In contrast, in the disinfectant of the present invention, even when the amount of each main component is trace, sufficient bactericidal activity can be obtained. This phenomenon has remarkable advantages in economy and safety because a lower concentration provides a higher effect.

Hereinafter, Tables 6-1 and 6-2 show examples of the effect of the disinfectant of the present invention in which L-cysteine is added into a mixture of various metal ions, L-ascorbic acid, and a surfactant, and the whole was adjusted to acidic (pH 3.0) with diluted hydrochloric acid.

[Table 6-1]

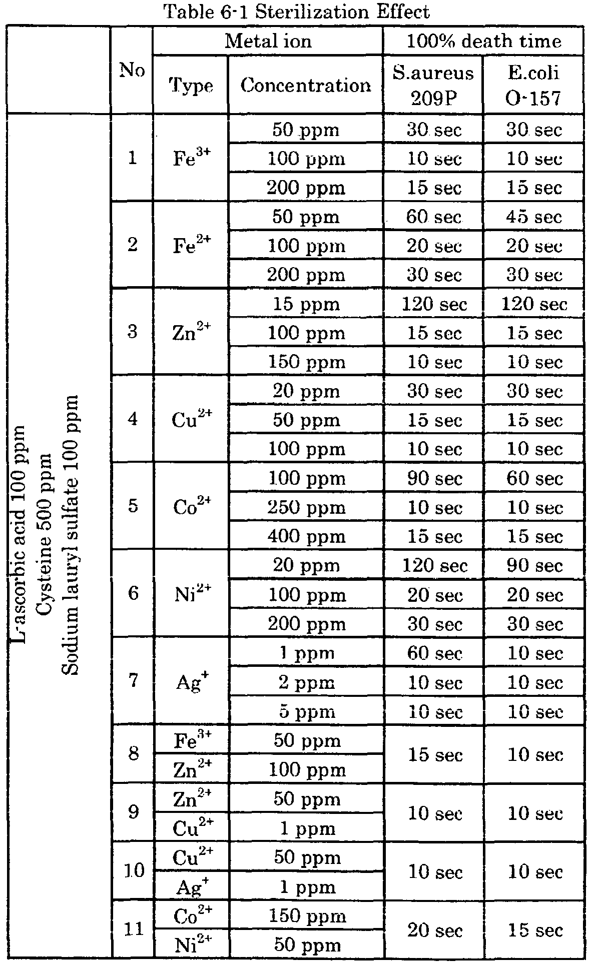

[Table 6-2]

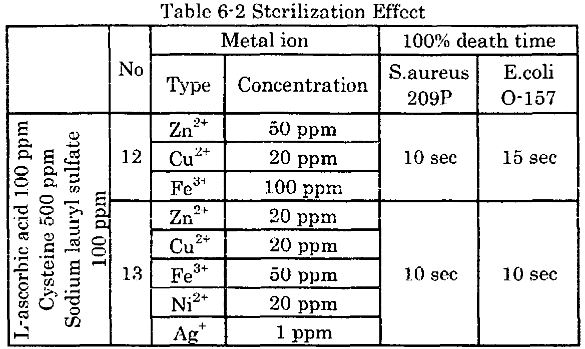

The results in Tables 6-1 and 6-2 reveal that, in an acidic aqueous solution (pH 3.0) into which a metal ion solution having a concentration in a range from the concentration at which strong antiproliferative activity is added to the concentration at which bacteriostasis is observed by the metal ion alone, L-cysteine, L-ascorbic acid, and a surfactant (sodium lauryl sulfate) are added so as to have concentrations 500 ppm, 100 ppm, and 100 ppm, respectively, sample bacteria are killed by 10 to 15 seconds contact in the optimum concentration of the metal ion.
Here, the optimum concentration of a metal ion slightly varies depending on the amount added of L-cysteine or the like.

As the next step, suspensions of various sample bacteria were prepared (about 1 × 10⁹ cells/physiological saline), each bacteria suspension was added to the disinfectant of the present invention shown in Table 6, No. 13 so as to be 2% by weight. The mixture was collected with a platinum loop with time and inoculated into each growth medium. The bacteria were cultured in an optimum environment, and the sterilization effect was determined by the proliferation of bacteria. The results are listed in Tables 7-1 and 7-2.

[Table 7-1]

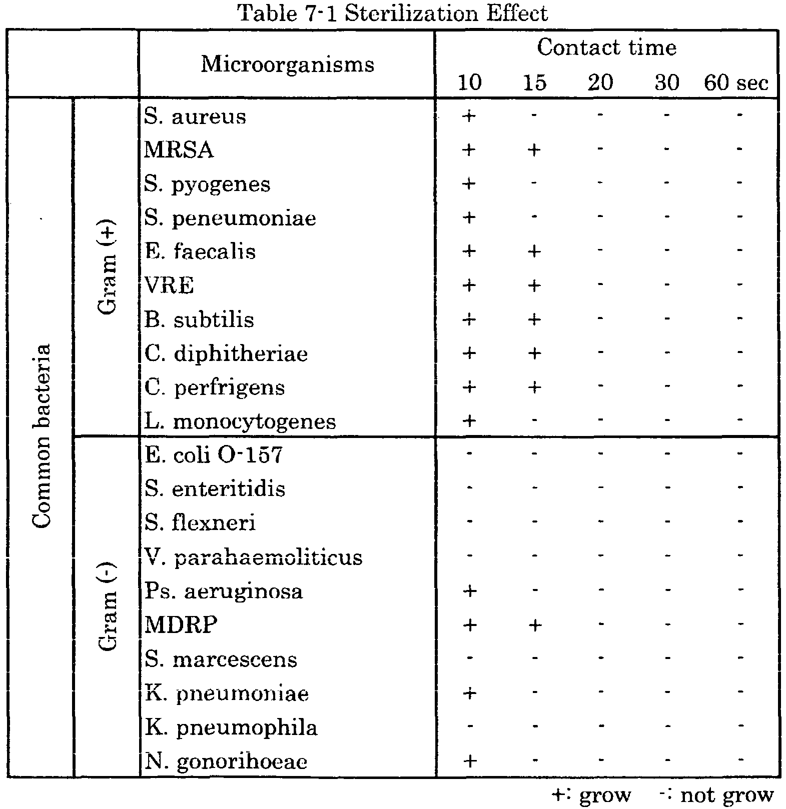

[Table 7-2]

**Table 7-2 Sterilization Effect**

| | Microorganisms | Contact time | | | | | |
|---|---|---|---|---|---|---|---|
| | | 10 | 15 | | 20 | 30 | 60 sec |
| Acid fast bacteria | M. tuberclosis | - | - | | - | - | - |
| | XDR-TB | - | - | | - | - | - |
| | M. kansasi | - | - | | - | - | - |
| | M. abium | - | - | | - | - | - |
| Fungi | C. albicans | + | + | | - | - | - |
| | Trichophyton | + | - | | - | - | - |
| | Aspergillus | + | - | | - | - | - |
| | Cryptococcus | + | + | | - | - | - |
| Spores | B. subtitis | + | | ~ | | - | - |
| | B. natto | + | | ~ | | - | - |
| | C. perfrigens | + | | ~ | | - | - |
| | C. tetani | + | | ~ | | - | - |
| Viruses | Avian influenza virus H5N | Activity was reduced to 1/100,000 to 1/1,000,000 by 5 minutes contact. Almost destroyed by 30 minutes contact | | | | | |
| | Norovirus | | | | | | |

| | | | | | | | |
|---|---|---|---|---|---|---|---|
| +: grow -: not grow | | | | | | | |

Apparent from Tables, the disinfectant could kill common bacteria within 10 seconds to 15 seconds and even acid fast bacteria within 1 minute. Furthermore, in the case of fungus, it could kill filamentous fungus within about 15 seconds and yeast fungus within about 30 seconds.

It could destroy spores within 1 minute to 120 minutes depending on their formation phase. (The details will be described in Example 12)

In the case of viruses, the disinfectant could deactivate avian influenza virus H5N3 with an envelope by 5 minutes contact and norovirus without an envelope by 30 minutes contact. (The details will be described in Example 13)

The disinfectant of the present invention may include a trace amount of essential oils and antimicrobial components derived from various plants such as Japanese cypress and mint, and antimicrobial components derived from mineral resources for enhancing the antimicrobial activity.

The action mechanism in the disinfectant of the present invention is complicated and has not been sufficiently identified, but it is suggested that the surface tension is decreased to cause trouble to cell membranes and to destroy them, and subsequently caused antimicrobial activity of metal ions (its main mechanism is strong oxidizing power, ion catalysis generates active oxygen, ion reduction generates hydrogen peroxide, the generated OH radical destroys microorganisms themselves, protein is solidified and denatured to cause trouble to an enzyme system, metabolic function is inhibited, further, the binding to an -SH group, a -COOH group, an -OH group, or the like in bacteria destroys cell nuclear membranes in the bacteria) is provided to the maximum extent to provide the killing activity for an extremely short period.

In the specification of the present invention, the disinfection means killing pathogens and is not concerned with nonpathogenic microorganisms. In contrast, the sterilization means killing not only pathogenic microorganisms but also all microorganisms. The disinfectant of the present invention means a disinfectant having either the disinfection activity or the sterilization activity. Furthermore, the "antimicrobial" means from the inhibition of microorganism proliferation to the killing of microorganisms.

The pathogens in the present invention means microorganisms such as bacteria and viruses that cause diseases. Examples of the pathogen include causative bacteria of various infections such as an intestinal infection, a respiratory tract infection, and a urinary tract infection. Examples of the causative bacteria include Salmonella spp., Shigella spp., Vibrio parahaemolyticus, Vibrio choreae, Escherichia coli 0-157, Campylobacter jejuni, Clostridium difficile, Clostridium perfringens, Yersinia enterocolitica, Helicobacter pylori, Entemoeba histolytica, Bacillusu cereus, Staphilococcus spp., Clostridium botulinum, Haemophilus influenzae, Streptococcus pneumoniae, Chlamidia pneumoniae, Legionella pneumoniae, Branhamella catarrhalis, Mycobacterium tuberculosis, Mycoplasma pneumonia, Storeptcoccus pyogenes, Corynebacterium diphtheriae, Bordetella pertussis, Chramidia psittaci, Pseudomonas aerginosa, methicillin resistant Staphylococcus aureus, MRSA, Escherichia coli, Klebsiella pneumoniae, Enterobacter spp., Proteus spp., Acinetobacter spp., Enterococcus faecalis, Staphylococcus saprophyticus, and Storeptcoccus agalactiae.
Furthermore, examples of the virus in the present invention include avian influenza virus, norovirus, hepatitis virus, AIDS virus, and rotavirus.

The disinfectant of the present invention can sterilize an object to be treated by bringing the disinfectant into contact with the object to be treated.

Examples of the object to be treated include an environment, an instrument, a human body, an animal body, a plant body, and an organic substance.

Examples of the environment include home environments such as a bedroom, a living room, a rest room, a toilet, and a bathroom; environments in transport machineries such as an automobile, a train, an aircraft, and a ship; special environments requiring cleanliness such as an operating room, a hospital room, and a resting room; environments for raising livestock such as a cat, a rabbit, a dog, a chicken, a sheep, a goat, a pig, a cow, and a horse; and aquafarms.
The environments further include furniture, tools, instruments, play equipments, and apparatuses present in the environments exemplified above.

Examples of the instrument include metal instruments, non-metal instruments, and their composite instruments.

Examples of the human body and the animal body include skins, fingers, and mucosae that are usually in contact with outside, as well as wound areas and diseased and lesion areas.

Examples of the plant body include vegetables, fruits, and ornamental plants.

Examples of the organic substance include blood, body fluid, sputum, pus, and excrement of human beings and animals.

The contact method of the disinfectant may be any conventional method such as spray and coating. When the disinfectant is sprayed into air, the air can be sterilized.

Hereinafter, specific preparation examples and examples will be explained, but the present invention is not intended to be limited to the examples.

### Examples

Various disinfectants can be prepared by the combination of the components of the present invention and their concentrations. However, only typical examples are described here and are used in the examples described later.
The basic preparation method is preferably a method in which a solution A containing a metal ion and a solution B containing components other than the metal ion are prepared separately, and the solutions are mixed (The solution A + the solution B = a predetermined concentration of each component). The volume of the solution A may be equal to that of the solution B, but the volume ratio of the solution A and the solution B is preferably about 1:9 to 2:8 because such ratio can prevent troubles such as precipitation of the components. When precipitated, the precipitate is filtered out to give a clear disinfectant with little effect on the sterilizability. In order to adjust pH, diluted hydrochloric acid, diluted sulfuric acid, or diluted nitric acid is preferably used after mixing.

### <Preparation Example 1>

Solution A: 0.96 g of ferric chloride hexahydrate was dissolved in 200 ml of purified water.

Solution B: 1 g of L-cysteine, 0.1 g of L-ascorbic acid, 0.05 g of potassium sorbate, and 0.5 g of sodium lauryl sulfate were dissolved in 800 ml of purified water.

Next, the solution A and the solution B were mixed, and 1 ml of 3N hydrochloric acid was added to prepare each disinfectant including the components described below.

Components: a Fe³⁺ ion 200 ppm, L-cysteine 1000 ppm, L-ascorbic acid 100 ppm, potassium sorbate 50 ppm, and sodium lauryl sulfate 100 ppm (pH 3.0).

### <Preparation Examples 2 to 10>

Hereinafter, various disinfectants of the present invention were prepared in a similar preparation method to that in Preparation Example 1, and are listed in Tables 8-1 and 8-2 as Preparation Examples 2 to 10.

[Table 8-1]

**Table 8-1 Preparation Example of the Disinfectant**

| Preparation No. | Mineral component | | Concentration of L**-**cysteine | Concentration of L-ascorbic acid | Type and concentration of surfactant | Type and concentration of food preservative | P11 |
|---|---|---|---|---|---|---|---|
| | Compound | Ion concentration | | | | | |
| 2 | ZnSO₁ 7H₂O | Zn²⁰ | 500ppm | 100 ppm | Alkylbenzene sulfonate | | 3.5 |
| | 0.66 g | 150 ppm | | | 100 ppm | | |
| 3 | CuSO_{4·}5H₂O | Cu²⁴ | 100 ppm | 100 ppm | Sodium lauroyl sarcosinate | | 3.0 |
| | 0.24 g | 60 ppm | | | 100 ppm | | |
| 4 | CoCl₂-6H₂O | Co²⁺ | 500 ppm | 300 ppm | | | 4.0 |
| | 0.45 g | 100 ppm | | | Benzalkonium chloride | Sodium benzoate | |
| | NiSO₄· 7H₂O | Ni²⁺ | | | 100 ppm | 50 ppm | |
| | 0.48 g | 100 ppm | | | | | |
| 5 | AgNO₃ | Ag²⁺ | 200 ppm | 100 ppm | Sodium lauroyl sarcosmate | | 3.0 |
| | 0.0032 g | 2 ppm | | | 100 ppm | | |
| 6 | FeCl₂ | Fc²⁺ | 1000 ppm | 100 ppm | | | 3.0 |
| | 0.68 g | 300 ppm | | | Benzothonum chloride | | |
| | AgNO₃ | Ag⁺ | | | 50 ppm | | |
| | 0.0024 g | 1.5 ppm | | | | | |
| 7 | ZnSO_{4·}7H₂O | Zn²⁺ | 800 ppm | 150 ppm | | | 3.5 |
| | 0.44 g | 150 ppm | | | Alkyldiaminoethylglycine hydrochloride | | |
| | FeCl_{2·}6H₂O | Fe³⁺ | | | 30 ppm | | |
| | 0.5 g | 100 ppm | | | | | |
| 8 | ZnSO₄·7H₂O | Zn²⁺ | 500 ppm | 100 ppm | Sodium lauryl sulfate | | 3.5 |
| | 0.44 g | 100 ppm | | | 50 ppm | | |
| | AgNO₃ | Ag⁺ | | | Polyoxyethylene sulfate alkyl ether sulfate | | |
| | 0.0026 g | 2 ppm | | | | | |
| | NiSO_{4·}7H₂O | Ni²⁺ | | | 50 ppm | | |
| | 0.48 g | 100 ppm | | | | | |

| | | | | | | | |
|---|---|---|---|---|---|---|---|
| (metal compound g/1000 ml = metal ion ppm) | | | | | | | |

[Table 8-2]

**Table 8-2 Preparation Example of the Disinfectant**

| Preparation No | Mineral component | | Concentration of L-cysteine | Concentration of L-ascorbic acid | Type and concentration of surfactant | Type and concentration of food preservative | PH |
|---|---|---|---|---|---|---|---|
| | Compound | Ion concentration | | | | | |
| 9 | FeCl_{3·}6H₂O | Fe²⁺ | 700 ppm | 100 ppm | | | 2.5 |
| | 0.24 g | 50 ppm | | | Higher alcohol sulfate | | |
| | Zn₃(C₆H₅O₉)₂· | Zn²⁺ | | | | | |
| | 2H₂O | 50 ppm | | | 50 ppm | | |
| | 0.16 g | Cu²⁺ | | | Benzalkonium chloride | | |
| | CuSO₄·5H₂O 5H ,O | 15 ppm | | | 50ppm | | |
| | 0.06 g | | | | | | |
| 10 | | Fe³⁺ | 1000 ppm | 100 ppm | | | 3 0 |
| | FeCl₃·6H₂O | 150 | | | | | |
| | 0.75 g | ppm | | | | | |
| | ZnSO₄·7H₂O | Zn²⁺ | | | Sodium lauryl sulfate | Potassium sorbate | |
| | 0.1 g | 23 ppm | | | 100 ppm | 50 ppm | |
| | NiSO₃·7H₂O | Ni²⁺ | | | | | |
| | 0.24 g | 50 ppm | | | | | |

| | | | | | | | |
|---|---|---|---|---|---|---|---|
| (metal compound g/1000 ml = metal ion ppm) | | | | | | | |

### <Example 1>

The activity of general-purpose disinfectants may be reduced in the presence of an organic substance. It is remarkably observed in sodium hypochlorite, quaternary ammonium salts, and biguanide disinfectants. The effect on the disinfectant of the present invention was examined using the solution prepared in Preparation Example 1.

Solutions of 0.1% and 1% chlorella extract (CGF) and solutions of 0.1% and 1% commercially available mixed fermented soybean paste were prepared. Then, each solution was added to the disinfectant so as to be 1% by amount or 5% by amount. Next, a suspension of each of S. aureus (MRSA) and E. coli O-157 (1 × 10⁹ cells/physiological saline) was inoculated at 1% by weight as sample bacteria. Then, 100 µL of the mixture was collected with time, inoculated into a medium at 37°C, and cultured. The sterilization effect was determined by the proliferation of bacteria, and the results are listed in Table 9.

[Table 9]

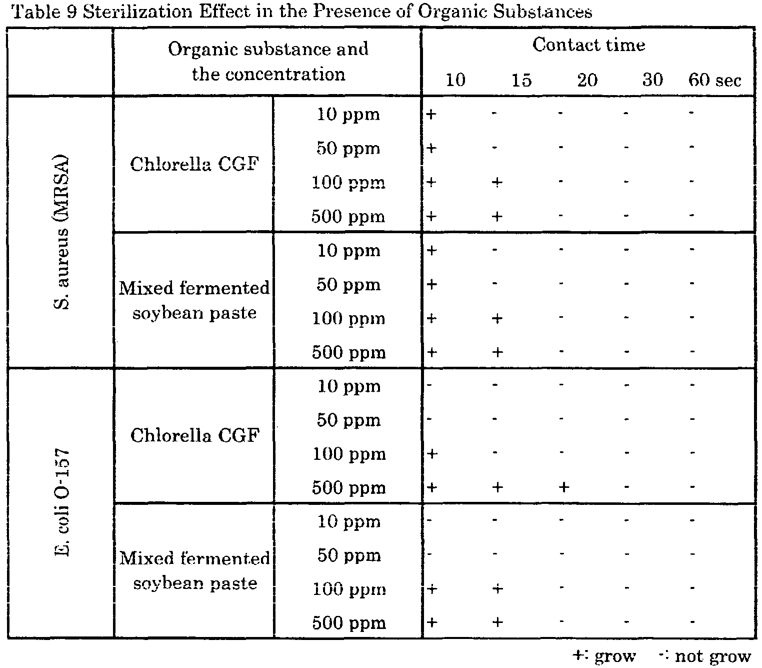

In the presence of the organic substances, when the concentration was low (50 ppm or less), the sterilization effect was not affected at all. However, when the concentration was 100 ppm or more, the efficacy was slightly decreased, but even when the concentration of the organic substance was 500 ppm, the bacteria was killed within 30 seconds. Thus, the deactivation observed in hypochlorites was not observed,

### <Example 2>

In the natural environment, bacteria actively proliferate in nutrient-rich foods, for example, to cause food poisoning. Effect of the disinfectant of the present invention was examined on such case. This would be the ultimate test of Example 1 in the presence of organic substances.

Each of boiled rice mixed with various ingredients (including carrot, fried thin tofu, burdock root, chicken, and hijiki), chilled tofu, Japanese omelet, sliced raw squid, and cooked pumpkin was left at 23°C for 24 hours. Then, onto the surface of each food, the disinfectant prepared in Preparation Example 9 was evenly sprayed. From immediately after the spray to 24 hours later, a part of each food was sampled and homogenized with a homogenizer. Then, the number of viable bacteria in one gram of each food was counted in a common procedure and are listed in Table 10.

As control solutions, water and a Hibitane solution (0.1% chlorhexidine gluconate solution) were sprayed.

[Table 10]

**Table 10 Sterilization Effect on Cooked Foods**

| Food | Water | The disinfectant | Hibitane solution |
|---|---|---|---|
| Boiled rice mixed with various ingredients *) | | | |
| Immediately after spray | 2×10⁶ | 5×10³ | 5×10⁵ |
| After 1 hour | 4×10⁶ | 10×10³ | 3×10⁴ |
| After 5 hours | 3×10⁷ | 7×10² | 1×10⁴ |
| After 24 hours | 2.5×10⁸ | 1×10² | 7×10⁴ |

| Chilled tofu *) | | | |
|---|---|---|---|
| Immediately after spray | 1×10⁸ | 3×10⁵ | 6×10⁶ |
| After 1 hour | 1.5×10⁸ | 5×10² | 5×10⁴ |
| After 5 hours | 5×10⁸ | 8×10 | 1×10⁵ |
| After 24 hours | 1.5×10⁹ | 5×10 | 8×10⁵ |

| Japanese omelet *) | | | |
|---|---|---|---|
| Immediately after spray | 5×10⁶ | 2×10² | 1×10³ |
| After 1 hour | 5×10⁶ | 2×10 | 5×10² |
| After 5 hours | 2×10⁷ | 0 | 5×10² |
| After 24 hours | 1×10⁸ | 0 | 1×10³ |

| Sliced raw squid *) | | | |
|---|---|---|---|
| immediately after spray | 3×10⁸ | 5×10⁴ | 2×10⁵ |
| After 1 hour | 4×10⁸ | 4×10³ | 7×10⁴ |
| After 5 hours | 1×10⁹ | 2×10² | 1×10⁵ |
| After 24 hours | 5×10⁹ | 2×10² | 6×10⁷ |

| Cooked pumpkin *) | | | |
|---|---|---|---|
| Immediately after spray | 5×10⁶ | 3×10⁴ | 5×10⁵ |
| After 1 hour | 5×10⁶ | 6×10² | 5×10⁵ |
| After 5 hours | 1×10⁷ | 5×10² | 1×10⁶ |
| After 24 hours | 8×10⁷ | 2×10² | 4×10⁷ |

| | | | |
|---|---|---|---|
| *) spray after 24 hours (the number of bacteria/g) | | | |

The rotting process of food naturally varies depending on food materials, cooking methods, and environments.

In the test, the number of viable bacteria in the boiled rice mixed with various ingredients was 2 × 10⁶/g at 24 hours after the cooking, and was increased to 2.5 × 10⁸/g at 48 hours after the cooking.

Similarly, in the chilled tofu, the number of viable bacteria was 1 × 10⁸/g at 24 hours after the cooking, and was increased to 1.5 × 10⁹/g at 48 hours after the cooking. The tofu had a slightly putrid smell.

In the Japanese omelet, the number of viable bacteria was 5 × 10⁶/g at 24 hours after the cooking, and was increased to 1 × 10⁸/g at 48 hours after the cooking. However, in a sensory test, no special abnormal event was observed.

In the sliced raw squid, the number of viable bacteria was 3 × 10⁸/g at 24 hours after the cooking, and the squid had a comparatively strong putrid smell. At 48 hours after the cooking, the number of viable bacteria was increased only ten times higher (5 × 10⁹/g), but the squid had a strong putrid smell.

In the case of the cooked pumpkin, the number of viable bacteria was 5 × 10⁶/g at 24 hours after the cooking, and was increased to 8 × 10⁷/g at 48 hours after the cooking. However, in the sensory test, the result seemed almost the same as that immediately after the cooking. (Hereinbefore, only water was sprayed)

As mentioned above, when the disinfectant was sprayed to a food that was slightly rotten, the number of viable bacteria was rapidly decreased to 1/1,000 to 1/10,000 immediately after the spray, to 1/10,000 to 1/1,000,000 after 1 hour, to 1/100,000 to 1/10,000,000 after 5 hours, and to 1/1,000,000 to 1/100,000,000 after 24 hours. The complete sterilization was not achieved except for the Japanese omelet. However, in such marked rotting process, the disinfectant provided sufficient sterilization effect.

In contrast, in the case of the Hibitane solution, the number of viable bacteria was decreased to 1/10 to 1/1,000 immediately after the spray, and continued to be decreased after 1 hour. The decreasing trend was stopped after 5 hours. After that, the number of viable bacteria started to be increased, and was considerable after 24 hours. Thus, the true sterilization effect was not observed.

### <Example 3>

General-purpose disinfectants have no effect on excrements (specimen) or weak effect even if they have, and usually have a notice of unsuitable for use. Thus, the efficacy of the disinfectant was examined. As control agents, 70% ethanol, a sodium hypochlorite solution (available chlorine 400 ppm), and a Hibitane solution were used.

### (1) Sputum

Sputum was collected from five persons, mixed, and pretreated with a sputum resolvent Sputasol. A part of the treated sputum was collected, and the number of viable bacteria was counted in a common procedure. To the residual specimen, 2 volumes of each disinfectant was added, and the number of viable bacteria in the specimen was counted with time. Here, the disinfectant prepared in Preparation Example 5 was used. (The same solution was used for feces and pus as the below specimens)

### (2) Feces

In collected feces, measurable intestinal bacteria were observed at 1.5 × 10¹¹/g. To the feces, 5 volumes of each of the various disinfectants was added, and the whole was well stirred. The number of viable bacteria in the feces was counted by anaerobic culture with time.

### (3) Pus

From bedsore patients, pus mainly including antibiotic resistant MRSA and pus mainly including antibiotic resistant Pseudomonas aeruginosa (MDRP) were collected. The number of viable bacteria per 1 g of each pus was counted. Next, 2 volumes of the disinfectant was added, and the number of viable bacteria in each specimen was counted with time in a common procedure.

Each sterilization effect on the above specimens (1), (2), and (3) is shown in Table 11.

[Table 11]

**Table 11 Sterilization Effect on Specimen (Excrement)**

| Specimen | Time after addition | The number of viable bacteria/g in specimen | | | |
|---|---|---|---|---|---|
| | | The disinfectant of the present invention | 70% Ethanol | Sodium hypochlorite | Hibitane solution |
| Sputum 2 × 10⁶/g | 5 min | 5×10⁵ | 1×10⁸ | 2×10⁴ | 5×10⁵ |
| | 15 min | 1×10³ | 2×10⁷ | 1×10⁴ | 1×10⁵ |
| | 30 mn | 0 | 6×10⁶ | 1×10⁴ | 1×10⁵ |
| | 60 min | 0 | 5×10⁶ | 5×10³ | 8×10⁴ |
| Feces 1.5×10¹¹/g | 5 min | 1×10⁹ | 5×10⁶ | 1×10⁸ | 8×10¹⁰ |
| | 15 min | 5×10⁴ | 3×10³ | 8×10⁶ | 5×10¹⁰ |
| | 30 min | 3×10³ | 4×10¹⁰ | 8×10⁶ | 5×10¹⁰ |
| | 60 min | 1.5×10² | 4×10¹⁶ | 1×10⁷ | 5×10¹⁰ |
| Pus (MRSA) 4 × 10⁹/g | 5 min | 5×10⁵ | 5×10⁷ | 3×10⁴ | 3×10⁶ |
| | 15 min | 2×10³ | 2×10⁵ | 5×10² | 4×10⁴ |
| | 30 min | 7×10 | 8×10⁴ | 1×10² | 6×10² |
| | 60 min | 0 | 5×10⁴ | 0 | 5×10² |
| Pus (MDRP) 5 × 10⁹/g | 5 min | 8×10⁵ | 7×10⁷ | 2×10³ | 5×10⁶ |
| | 15 min | 4×10² | 5×10⁵ | 1×10³ | 5×10⁶ |
| | 30 min | 0 | 1×10⁴ | 2×10² | 2×10² |
| | 60 min | 0 | 1×10⁴ | 1×10² | 3×10² |

In the sputum, the number of viable bacteria was 2 × 10⁹/g. When 2 volumes of the disinfectant of the present invention was added, the number was decreased to one-several thousands after 5 minutes, and to 1/2,000,000 after 15 minutes. After 30 minutes, the bacteria were completely killed.
In contrast, in the case of 70% ethanol, after 60 minutes, the number of viable bacteria was decreased only to 1/1,000 and was as many as 5 × 10⁶/g. In the case of sodium hypochlorite, the number of viable bacteria was rapidly decreased after addition, but continued at almost the same level, and was 5 × 10³/g even after 60 minutes. The Hibitane solution had a similar trend to that of the hypochlorite, and the number of viable bacteria was 8 × 10⁴/g even after 60 minutes.
Next, in the feces, the number of measurable viable bacteria was 1.5 × 10¹¹/g. However, when 5 volumes of the disinfectant of the present invention was added, the number was decreased to 1/100 after 5 minutes and to 1/2,000,000 after 15 minutes. Even after 60 minutes, the bacteria were not completely killed, but the number was about 150/g. Thus, almost complete sterilization may be achieved.
In contrast, by 70% ethanol and the Hibitane solution, the bacteria were only slightly decreased. Thus, they are proved to have no effect. By the hypochlorite, the bacteria were decreased to about 1/10,000 after 60 minutes (1 × 10⁷/g). It has little effect.
In the case of the pus, the result from the pus mainly including MRSA did not have significant difference from that from the pus mainly including MDRP, and each tested agent had the effect.
That is, by the disinfectant of the present invention, the bacteria were completely killed after 30 minutes. By the general-purpose disinfectants, the bacteria were not completely killed by 60 minutes contact, but the number of the bacteria was drastically decreased to 1 × 10² to 5 × 10⁴/g_{.}

### <Example 4>

Next, the efficacy of the disinfectant was examined when it is used for a gargle. The number of viable bacteria was counted in 1 ml of saliva that was collected from each of three persons before a gargle. Then, each person gargled three times each for 20 seconds, that is, gargled for total of 1 minute, with the disinfectant of the present invention that was prepared in Preparation Example 6. Each saliva was collected immediately after the gargle and at 15 minutes, 30 minutes, and 60 minutes after the gargle. The number of viable bacteria was counted per 1 ml of the saliva, and the effect of gargle was determined. Here, the number of viable bacteria before gargle is regarded as 100, and relative values are shown.

As a control, each of two persons gargled with tap water or 1000 ppm povidone-iodine in a similar way. The results are shown in Table 12.

[Table 12]

**Table 12 Effect of Gargle**

| | | The disinfectant of the present invention 3 persons | | | Tap water 2 persons | | Povidone-iodine 2 persons | |
|---|---|---|---|---|---|---|---|---|
| The number of viable bacteria before gargle | | 100 | 100 | 100 | 100 | 100 | 100 | 100 |
| 3 times gargles | Immediately after gargle | 2 | 1 | 2 | 5 | 10 | 2 | 2 |
| | After 15 min | 3 | 2 | 5 | 10 | 25 | 5 | 8 |
| | After 30 min | 10 | 8 | 12 | 20 | 40 | 18 | 20 |
| | After 60 min | 20 | 15 | 15 | 50 | 70 | 30 | 40 |

The gargle even with tap water reduced the number of bacteria to 5 to 10% immediately after the gargle. However, it is revealed that the number returns to the original level unexpectedly sooner with time.
On the other hand, in the case of povidone-iodine that is recommended as a gargle, the number of bacteria was reduced to 2% immediately after the gargle, but after that, was gradually increased, and after 60 minutes, returned to 30 to 40%. Thus, the result was not largely different from that of the gargle with tap water.
In contrast, when the disinfectant of the present invention was used, the number of bacteria was 1 to 2% immediately after gargle, 2 to 5% after 15 minutes, 8 to 12% after 30 minutes, and 15 to 20% even after 60 minutes. It is clear that the effect of gargle continues for a long time. There are some reports that chronic gingivitis or periodontal disease was reduced or completely treated while gargle with the disinfectant had been continued.

### <Example 5>

The sterilization effect was examined when the disinfectant was widely applied on hand skin.

The disinfectant prepared in Preparation Example 3 was applied. Immediately after that, an area of 10 cm² was wiped with sterile gauze that was immersed into sterile physiological saline. Then, the number of bacteria was counted (Swab method). Next, different areas each having an area of 10 cm² were wiped in the applied area after 15 minutes, after 30 minutes, and after 60 minutes, and the viability of bacteria on the skin was determined. As controls, tap water and 70% ethanol were used. The number of bacteria before the application of disinfectants is regarded as 100 and relative values are shown. These results are shown in Table 13.

[Table 13]

**Table 13 Decrease of Bacteria after Application of Disinfectants**

| Elapsed time | The number of viable bacteria on skin surface | | |
|---|---|---|---|
| | The disinfectant of the present invention | Tap water | 70% Ethanol |
| before gargle | 100 | 100 | 100 |
| Immediately after gargle | 0 | 85 | 0 |
| After 15 min | 0.01 | 95 | 12 |
| After 30 min | 0.01 | 100 | 20 |
| After 60 min | 0.04 | 100 | 45 |

As apparent from Table, the tap water naturally has no bactericidal activity, and the result was within the margin of error. In the case of 70% ethanol, no bacteria was observed immediately after application, then the bacteria gradually appeared (due to falling bacteria or transferred bacteria), and after 60 minutes, the number of bacteria returned to 45% of that before application.
In contrast, in the case of the disinfectant of the present invention, no bacteria were observed immediately after application, the detection rate was 0.01% after 30 minutes, and was only 0.04% even after 60 minutes. Thus, it is proved that the efficacy continues.

### < Example 6>

The efficacy was examined on doorknobs (stainless-steel) and urinals that are readily contaminated with bacteria or fungi by contact of an indefinite number of persons in a hospital. They were used in a usual way immediately after cleansing, and then the viability of adhered bacteria and the contamination were observed with time. The results are shown in Table 14.

Here, the disinfectant of the present invention prepared in Preparation Example 7 was used, and tap water was used as a control.

[Table 14]

**Table 14 Sterilization Effect on Instruments**

| Instrument | Elapsed time | The number of viable bacteria adhered on an instrument/10 cm2 | |
|---|---|---|---|
| | | The disinfectant of the present invention | Tap water |
| Doorknob | Before cleansing | 100 | 100 |
| | Immediately after cleansing | 0 | 0.1 |
| | After 1 hour | 0 | 0.5 |
| | After 6 hours | 0.01 | 2 |
| | After 24 hours | 0.2 | 55 |
| | After 48 hours | 1 | 98 |
| Urinal | Before cleansing | 100 | 100 |
| | Immediately after cleansing | 0 | 0.5 |
| | After 1 hour | 0.2 | 2 |
| | After 6 hours | 1 | 15 |
| | After 24 hours | 10 | 85 |
| | After 48 hours | 20 | 100 |
| | Contamination after 48 hours | A pale yellow substance was partly adhered, but was cleaned only by water flushing | Contaminated with a yellow substance and partly accumulated. Not cleaned only by water flushing |

| | | | |
|---|---|---|---|
| * The number of bacteria before cleansing is regarded as 100 | | | |

Bacteria adhered on the knob before cleansing were completely killed by the disinfectant of the present invention immediately after cleansing. The effect continued several hours. The number of bacteria returned only to 1/10,000 of the original level even after 6 hours, to 1/500 after 24 hours, and to 1/100 even after 48 hours. It is also proven here that the efficacy continues for a long time.
In contrast, in the case of the tap water, the number of bacteria was reduced to 1/1,000 immediately after cleansing. However, after that, the number of adhered bacteria continued to be increased, and was about 1/2 of the original level after 24 hours, and returned to the original level after 48 hours.
In the test on urinals, the efficacy was not so much because of nutrient resupply (urine) as that on the knob. However, the number of bacteria was reduced to 1/10 of that before cleansing even after 24 hours, and to 1/5 even after 48 hours. The tap water had the same trend as that in the knob, and the number of bacteria returned to the original level after 48 hours.

### <Example 7>

To a floor (plastic tile) having a lot of contamination, the disinfectant prepared in Preparation Example 8 was sprayed. Different areas each having an area of 100 cm² were wiped with wet sterile gauze with time. Each number of bacteria was counted, and the viability is shown in Table 15. Tap water was used as a control.

[Table 15]

**Table 15 Efficacy of Disinfectant on Floor**

| Elapsed time | The number of viable bacteria on floor | |
|---|---|---|
| | The disinfectant of the present invention | Tap water |
| before spray | 100 | 100 |
| Immediately after spray | 0.07 | 100 |
| After 15 min | 0 | 80 |
| After 30 min | 0 | 100 |
| After 60 min | 0.02 | 100 |

| | | |
|---|---|---|
| The number of bacteria immediately after spray: 100 | | |

In the case of tap water, a certain number of bacteria were observed without sterilization. In contrast, when the disinfectant was sprayed, viable bacteria were slightly observed immediately after spray, but no bacteria were observed after 15 minutes and after 30 minutes, and trace bacteria were observed after 60 minutes.
This means that in the condition that a floor is wet with the disinfectant, even when bacteria are adhered, they are immediately sterilized.

### <Example 8>

Air in a closed room having an area of about 13 m² was collected with a syringe having a volume of 100 ml. Microorganisms in the air were classified into common bacteria, spores, and fungi, and the viable number of each group was counted.

Next, into a tank of a commercially available ultrasonic wave spray device Micro Fogger (manufactured by Aquamide, UV-200SP), the disinfectant of the present invention prepared in Preparation Example 9 was poured. The disinfectant was sprayed at 20 mL/min (particle size: 25 µm). Then, air was collected at a certain place with time with a syringe having a volume of 100 ml. The number of viable bacteria contained was counted, and the ratios are listed in Table 16.

[Table 16]

**Table 16 Ratio of the Number of Viable Bacteria in Room Environment**

| Microorganism type | Before spray | Ratio (%) of the number of viable bacteria after spray | | | | |
|---|---|---|---|---|---|---|
| | | 5 min | 15 min | 30 min | 60 min | 120 min |
| Common bacteria | 100 | 5 | 2 | 1 | 0 | 0 |
| Spores | 100 | 100 | 75 | 30 | 10 | 1 |
| Fungi | 100 | 35 | 15 | 8 | 0 | 0 |

As apparent from Table, the number of viable common bacteria was remarkably decreased to 5% after 5 minutes of spray and to 1% after 30 minutes. After 60 minutes, the common bacteria were completely killed.
The number of viable fungi was decreased to 35% after 5 minutes of spray and to 8% after 30 minutes. After 60 minutes, the fungi were completely killed. On the other hand, the spores were not affected at all after 5 minutes. However, after that, the number of spores was gradually decreased, and the spores were almost decayed after 120 minutes.
(Viability: 1%)

This means that when not an open room, continuous spray of particles of the disinfectant can almost sterilize a room. Thus, a new type of high-performance sterilization air cleaner (bacteria- and virus-free) may be developed.

### <Example 9>

(1) It is not known unexpectedly that vegetables have a proportion of about 30% in food materials causing food poisoning. When raw vegetables are eaten, it is important for preventing food poisoning to sufficiently wash the vegetables with running water, also from the viewpoint of residual pesticides. Here, into the disinfectant prepared in Preparation Example 2, each vegetable (cabbage, white radish sprouts, and lettuce) was immersed for 2 minutes and next washed with tap water for 15 seconds. Then, the number of adhered viable bacteria was counted per unit area of each vegetable with time. As controls, vegetables that were washed with running water alone for 15 seconds were used. These results are shown in Table 17.

[Table 17]

**Table 17 Sterilization and Washing Effect on Vegetable**

| | | The number of viable bacteria/cm² on vegetable surface | |
|---|---|---|---|
| | | The disinfectant and tap water | Tap water |
| Cabbage | Before washing | 2×10⁴ | 5×10⁴ |
| | Immediately after washing | 0 | 6×10 |
| | After 1 hour | 5 | 4×10² |
| | After 6 hours | 5×10 | 5×10³ |
| White radish sprouts | Before washing | 4×10⁴ | 5×10⁴ |
| | Immediately after washing | 0 | 1×10² |
| | After 1 hour | 2×10 | 1×10³ |
| | After 6 hours | 1×10² | 5×10⁴ |
| Lettuce | Before washing | 3×10³ | 8×10² |
| | Immediately after washing | 0 | 1×10 |
| | After 1 hour | 0 | 5×10 |
| | After 6 hours | 3×10 | 2×10² |

It is ascertained that all of the sample vegetables are sterile immediately after the immersion for 2 minutes in the disinfectant, that, after that, the number of viable adhered bacteria is small, and that, in contrast, simple washing with running water simply removes bacteria.

(2) It is well known that fishes are relatively quickly rotten due to a large number of pathogens and putrefactive bacteria which are present not only on surfaces of fishes but also inside their scales, and are typical food materials causing food poisoning. In particular, the Japanese like to eat law fishes, and thus such events often occur. Here, an examination was carried out on mackerels and sardines that are extremely quickly rotten. The disinfectant prepared in Preparation Example 5 was poured in a container. A fish body was placed into the container and immersed for 1 minute. Then, the surface of the fish body was cleaned with light scrubbing in running water for 20 seconds, and the fish body was left at room temperature (23°C).

A fish body washed in running water alone for 20 seconds was used as a control.

The detected bacteria were classified into Vibrio species that are comparatively common in fishes and other species. The number of viable bacteria before washing and the number of viable bacteria after washing with time were counted, and the results are listed in Table 18.

[Table 18]

**Table 18 Sterilization and Washing Effect on Fish**

| | | The number of viable bacteria/cm² on fish surface | | | |
|---|---|---|---|---|---|
| | | The disinfectant and tap water | | Tap water | |
| | | Vibrio species | Common bacteria | Vibrio species | Common bacteria |
| Mackerel | Before washing | 8×10² | 2×10⁵ | 5×10² | 1×10⁵ |
| | After washing | 0 | 0 | 2×10 | 5×10² |
| | After 1 hour | 0 | 1×10 | 1×10² | 3×10³ |
| | After 6 hours | 0 | 5×10² | 4×10³ | 3×10⁶ |
| Sardine | Before washing | 1×10³ | 1.5×10⁶ | 3×10² | 2×10⁵ |
| | After washing | 0 | 0 | 2×10 | 2×10² |
| | After 1 hour | 0 | 2×10 | 5×10 | 1×10³ |
| | After 6 hours | 0 | 3×10² | 2×10³ | 5×10⁵ |

Not depending on the fish type, the number of adhered bacteria was rapidly decreased to 1/200 to 1/1,000 immediately after washing with running water. However, the bacteria gradually proliferated by leaving, and after 6 hours, the number of bacteria was larger than that before washing. In particular, the Vibrio species were remarkable, and the number was increased to about 10 times. The reason is unknown but may relate to mucus removal condition from the surface of fish body.
In contrast, immediately after the immersion in the disinfectant for 1 minute and taking out, neither the common bacteria nor the vibrio species were detected. Even after 6 hours, the number of bacteria was kept 1/20 to 1/500 of that before washing (The detected bacteria were mainly due to falling bacteria).
That is, as with vegetables, the sterilization effect continues for a long time, and the disinfectant may prevent the food poisoning caused by fishes. The disinfectant did not change the flavors and tastes of vegetables and fishes at all and kept the original delicious tastes.

### <Example 10>

Among pets raised in home, the washing effects were examined on two guinea pigs and two terriers that are indoor dogs. Into tap water and the disinfectant prepared in Preparation Example 4 each warmed at 35°C, the whole body was immersed for 3 minutes while washing with light brushing. After washing, the body hair was dried with a drier. The number of bacteria was counted on the body surface immediately after drying and after 24 hours, and the hair condition and body odor were observed.

As a result, in the case of the guinea pig, the number of adhered bacteria was 1 × 10⁶/cm² after washing. By the washing with tap water, the number was decreased to 5 × 10⁴/cm², that is, to 1/20, but after 24 hours, the number of bacteria and the body odor returned to the level before washing.

In contrast, when the disinfectant was used, the number of bacteria was decreased to 2 × 10²/cm², that is, to 1/5000. After that, the number of bacteria gradually returned, but even after 24 hours, the number was kept at 1/250 (4 × 10³/cm²) of that before washing. The body hair had a sheen and was raised, and the body odor was slight.

In the case of the indoor dog, the number of bacteria adhered on body surface was about 1/10 of that of guinea pig. When washed with the disinfectant, the result was similar to that of the guinea pig, but the body odor was little even after 24 hours, and the effect continued for 72 hours (3 days).

### <Example 11>

### (1) Clinical Study Example 1

Clinical study was carried out for three months on eight patients with interdigital, bullous, or hyperkeratotic trichophytosis (athlete's foot) and two patients with trichophytosis unguium.

The clinical study was carried out in the following manner: an affected area was immersed for 15 minutes in the disinfectant prepared in Preparation Example 10 included in a resin container; and then, the affected area was washed with running water.

It is well known among specialists that the trichophytosis is hard to be completely cured, and that the trichophytosis unguium is especially hard.

Table 19 shows symptoms of trichophytosis of the clinical study patients, microscopic test results of skins and nails collected from the affected areas, and cultivation test results of the affected areas pretreated with potassium hydroxide.

[Table 19]

**Table 19 Clinical Study of Trichophytosis**

| Clinical study patien | | | Symptom and trichophytosis type | | | | Clinical study result after 3 months | |
|---|---|---|---|---|---|---|---|---|
| Name | Age | Sex | Type | Symptom | Trichophyton | | | Cultivation |
| | | | | | Microscopic test | Cultivation | | |
| A.A | 45 | ♀ | Interdigital | A surface of interdigital area was exfoliated | ++ | +++ | Almost completely cured | - |
| T.M | 65 | ♂ | Hyperkeratotic | A heel area was keratinized | + | ++ | The keratinized area was slightly softened | ┴ |
| S.N | 68 | ♀ | Interdigital | A surface of interdigital area was exfoliated | + | + | Almost invisible and almost completely cured | - |
| S.K | 37 | ♀ | Bullous | An interdigital area had blisters, and a nail was slightly thickened | - | + | The keratinized area was a little reduced, new epidermis appeared, almost completely cured | - |
| I.M | 40 | ♂ | Hyperkeratotic | A whole sole was keratinized | ++ | + | The keratinized area was a little reduced, but not completely cured | - |
| K.N | 24 | ♀ | Interdigital | An interdigital area had scales | + | ++ | Became smooth and almost completely cured | - |
| H.K | 65 | ♀ | Hyperkeratotic | An interdigital area was keratinized | + | ++ | Not improved | ± |
| K.K | 55 | ♂ | Bullous | An interdigital area had blisters | + | + | The busters usappeared, but not completely cured not | + |
| M.T | 42 | ♂ | Trichophytosis unguium | A nail was thickened and turned dark brown | +++ | ++ | Trichophyton was reduced, but gross features were not specifically changed | + |
| Y.F | 38 | ♂ | Trichophytosis unguium | A nail was thickened and a leading end was destroyed | + | + | A little thin nail grew from the root of the thickened nail | - |

Apparent from Table, the results of the clinical study varies depending on patients, and are from the almost completely cured level to the little improved level.
However, the cultivation features of Trichophyton after three months were almost negative except trichophytosis unguium. When the treatment is continued, it may be completely cured.

### (2) Clinical Study Example 2

Five volunteers with chronic sinusitis were treated with nasal irrigation every day for three months using the disinfectant prepared in Preparation Example 1. From the diagnosis by subjective symptoms and pathologic findings, in all five volunteers, symptoms were reduced after about over one month, and after three months, the relief of symptoms was ascertained by both pathological examination and X-ray examination. One of the clinical study volunteers reported the complete treatment by six months irrigation.

### (3) Clinical Study Example 3

For eliminating Helicobacter pylori that is known to be causative bacteria of gastric ulcer and duodenal ulcer and has a risk of stomach cancer when inflammation lasts for many years, three antibiotics are recommended to be administered for a week. At the present time, since resistant bacteria are increased, the elimination ratio is decreased to about 80 to 85%, and the eradication may become difficult gradually. In order examine the efficacy of the administration of the disinfectant of the present invention including, as main components, amino acids, vitamins, and minerals that are basic substances of life, clinical study was carried out on one of the inventors with Helicobacter pylori positive.

Sixteen ml of the solution prepared in No. 1 was diluted 3-fold, and about 50 ml of the diluted solution was administered once daily for a week. After an interval of one month, urea breath test was carried out again, and the result was negative. The disinfectant has a possibility of practical use for eliminating Helicobacter pylori.

### <Example 12>

Sands in a sandbox in a park and riverside soil were collected and mixed. The mixture was dried with heated air at 80 to 90°C for 6 hours. Then, 1 g of the dried mixture was added into water and immediately filtered with a filter. A part of the filtrate was applied onto a glass slide, and spores were stained in a common procedure. The number of stained spores was counted and the number of spores contained in 1 g of soil was determined. Simultaneously, the spores were aerobically (Bacillus species proliferated) and anaerobically (Clostridium species proliferated) cultured, and each ratio was determined.

Then, into 10 ml of the sample disinfectant, 1 g of the dried soil was added. Some of the mixture was collected with time, and cultured aerobically and anaerobically. The number of growing colonies was counted to determine the decay rate of the spores. These results are shown in Table 20.

[Table 20]

**Table 20 Spore Decay Time and Decay Rate**

| The disinfectant of the present invention | Spores of Bacillus species | | | | | | Spores of Clostridium species | | | | | |
|---|---|---|---|---|---|---|---|---|---|---|---|---|
| | 1 | 5 | 10 | 30 | 60 | 120 min | 1 | 5 | 10 | 30 | 60 | 120 min |
| Preparation Example 1 | 0 | 5 | 10 | 35 | 50 | 95% | 0 | 5 | 15 | 40 | 65 | 95% |
| Preparation Example 3 | 0 | 5 | 12 | 35 | 65 | 92% | 0 | 5 | 15 | 45 | 70 | 95% |
| Preparation Example 5 | 2 | 15 | 30 | 50 | 72 | 98% | 0 | 10 | 25 | 40 | 80 | 99% |
| Preparation Example 7 | 5 | 20 | 50 | 75 | 85 | 100% | 2 | 15 | 50 | 75 | 85 | 100% |
| Preparation Example 9 | 10 | 25 | 50 | 80 | 90 | 100% | 3 | 30 | 60 | 75 | 90 | 100% |
| 70% Ethanol | 0 | 0 | 0 | 0 | 0 | 0% | 0 | 0 | 0 | 0 | 0 | 0% |
| Sodium hypochlorite | 0 | 0 | 10 | 30 | 100 | 100% | 0 | 5 | 20 | 30 | 100 | 100% |

It was revealed that, in both of the Bacillus species and the Clostridium species, about 50% of their spores were decayed by 30 minutes contact of the disinfectant of the present invention, 70% or more of them were decayed by 60 minutes contact, and almost all spores were decayed by 120 minutes contact.
In contrast, 70% ethanol had no effect on the spores, and in the case of the hypochlorite (available chlorine: 400 ppm), 100% of spores were decayed by 60 minutes or more contact. However, when the contact time was short, the spores often were not decayed. Furthermore, the hypochlorite was quickly deactivated in some soil and often had no efficacy.

### <Example 13>

### Examination was carried out on avian influenza virus with an envelope.

Five strains of avian influenza virus, H3N2, H4N6, H5N3, H6N6, and H7H7 strains were prepared, and each was cultured in SPF embryonated eggs. Then, 0.1 ml of the virus solution was mixed with 0.5 ml of the disinfectant of the present invention prepared in Preparation Example 10. The mixture was left at room temperature for 10 minutes, and was serially diluted (10-fold steps) with a phosphate buffer solution (pH 7.2). Each 0.2 ml of the diluted solutions was inoculated into each allantoic cavity of five SPF embryonated eggs (10 days of age). Each egg was incubated at 37°C for 3 days. Then, the egg was left at 4°C overnight, and chicken erythrocyte hemagglutinating activity in the allantoic fluid was measured to determine the virus potency. As a control, a phosphate buffer solution (pH 7.2) was used.

As a result, the potency of each virus was decreased to 1/100,000 to 1/1,000,000 of the original value. Thus, it is ascertained that the disinfectant has very strong deactivation effect on the virus.

Furthermore, the test on norovirus without an envelope was carried out in a specialized agency. Oysters infected with the virus and feces from a person infected with the virus were immersed in 10 volumes of the disinfectant for 30 minutes, and the viability of virus was observed. As a result, the virus reaction was negative.

Hereinbefore, a part of the examples are introduced. Lastly, toxicity test will be described.

As for toxicity of the disinfectant of the present invention prepared in Preparation Example 10, LD50 in mice was 1 ml> by oral administration and 4 ml by intraperitoneal administration. Toxicity against animal cells (cytostatic activity) slightly varied depending on sample cells (monkey kidney CV-1 cells and human lymphocytes), but even when diluted 10-fold, about half cells were not affected to proliferate. When diluted 1,000- to 10,000-fold, no toxicity was observed.

In contrast, when a Hibitane solution (0.1% chlorhexidine gluconate) was diluted 10,000-fold, strong toxicity remained.

The disinfectants of the present invention prepared in other Preparation Examples have similar low toxicity to the above, and thus the disinfectant is proven to be nonconventional and highly safe.

In addition, differences in features between the disinfectant of the present invention and the disinfectant according to Patent Document 1 are listed in Table 21.

[Table 21]

**Table 21 Comparative Features of the Disinfectant of the Present Invention and the Disinfectant According to Patent Document 1**

| Desirable features for disinfectant | Evaluation result | |
|---|---|---|
| | The disinfectant of Patent Document 1 | The disinfectant of the present invention |
| (1) Broad antimicrobial spectrum | ++ | +++ |
| (2) Immediate efficacy | ++ | +++ |
| (3) Continuousness | + | +++ |
| (4) Sterilizing power in the presence of organic substances | + | ++ |
| (5) Low toxicity | +∼++ | +++ |
| (6) Easy handling | ++ | +++ |
| (7) Osmosis | + | +++ |
| (8) No unpleasant odor and taste | - | ++ |
| (9) Low cost | ++ | +++ |
| (10) Preservability | + | +++ |
| (11) Readily discarded | + | +++ |
| (12) No damage on an object to be treated | - | ++ |
| (13) Hard to generate resistant bacteria | ++ | +++ |

| | | |
|---|---|---|
| The results were obtained by evaluations in the same condition. In Table, the more the number of "+" is, the more preferred the features are | | |

The results in Table 21 reveals that the disinfectant of the present invention has more excellent features in various points than the disinfectant according to Patent Document 1 that the inventors have provided.
Among them, because the disinfectant of the present invention has the killing power to viruses as the sterilizing power, it has a broad antimicrobial spectrum. Furthermore, it provides remarkable effects of excellent continuous sterilizing power and excellent sterilizing power in the presence of organic substances. In addition, the disinfectant of the present invention has excellent osmosis to a substance with which the disinfectant is in contact, especially to microorganism cells and the like. It also has easy handling in the point of no unpleasant odors, excellent preservability, and less damage to an object. It further has excellent safety.

### Industrial Applicability

As described above, the disinfectant of the present invention is an extremely convenient disinfectant that is just the "versatile" as compared with conventional disinfectants. Its application is broad and unlimited. For example, it may be used for the prevention and containment of pandemic of a new type of highly pathogenic influenza mutated form avian H5N1 that seems a matter of time. Furthermore, it may be used for sterilization and disinfection and virus-free means that are absolutely necessary for increase of food production by biotechnology and for cutting-edge medical technology that fully used versatile cell.

While many advantages of the present invention included in the specification were described above, it will be understood that the disclosure is only an illustrative example in many aspects.

It is to be understood that the scope of the invention is limited to the appended claims.

## Claims

1. A disinfectant comprising:
a metal ion having antimicrobial activity, L-cysteine, and L-ascorbic acid as main components; and
in addition to the main components, a surfactant other than a non-ionic surfactant.

2. The disinfectant according to claim 1, wherein the metal ion having antimicrobial activity is a trivalent iron ion (Fe³⁺), a divalent iron ion (Fe²⁺), a zinc ion (Zn²⁺), a copper ion (Cu²⁺), a cobalt ion (Co²⁺), a nickel ion (Ni²⁺), or a silver ion (Ag⁺).

3. The disinfectant according to claim 2, wherein the metal ion having antimicrobial activity has a concentration of 50 to 200 ppm in the case of the trivalent iron ion, 110 to 400 ppm in the case of the divalent iron ion, 7.5 to 125 ppm in the case of the zinc ion, 15 to 60 ppm in the case of the copper ion, 180 to 300 ppm in the case of the cobalt ion, 85 to 175 ppm in the case of the nickel ion, and 1 to 3 ppm in the case of the silver ion.

4. The disinfectant according to any one of claims 1 to 3, wherein the L-cysteine has a concentration of 100 to 1000 ppm and the L-ascorbic acid has a concentration of 100 to 500 ppm.

5. The disinfectant according to any one of claims 1 to 4, wherein the surfactant other than a non-ionic surfactant is one or more surfactants selected from the group consisting of an alkylbenzene sulfonate, a linear alkylbenzene sulfonate, a polyoxyethylene alkyl ether sulfate, a higher alcohol sulfate, sodium lauryl sulfate, sodium lauroyl sarcosinate, stearyl dimethyl benzyl ammonium chloride, benzalkonium chloride, benzethonium chloride, alkyldiaminoethylglycine hydrochloride, and alkylpolyaminoethylglycine hydrochloride.

6. The disinfectant according to any one of claims 1 to 5, wherein the surfactant other than a non-ionic surfactant has a concentration of 20 to 100 ppm.

7. The disinfectant according to any one of claims 1 to 6, further comprising one or more compounds selected from the group consisting of sorbic acid, a sorbate, benzoic acid, a benzoate, and a para-oxybenzoate.

8. The disinfectant according to claim 7, wherein each of the sorbic acid, sorbate, benzoic acid, benzoate, and para-oxybenzoate has a concentration of 50 to 100 ppm.

9. The disinfectant according to any one of claims 1 to 8, wherein the disinfectant is adjusted to pH 2.5 to 4.0.

10. A sterilization method comprising:
bringing the disinfectant according to any one of claims 1 to 9 into contact with an environment, an instrument, a plant body, or an organic substance.

11. The disinfectant according to any of claims 1 to 9 for use in the adjunctive treatment of infections in human beings and animals.

12. The disinfectant according to any one of claims 1 to 9 for use in the disinfection or sterilization of the skin or mucosa of a human or animal body.

## Patentansprüche

1. Desinfektionsmittel umfassend:
ein Metallion, das anti-mikrobielle Aktivität besitzt, L-Cystein und L-Ascorbinsäure als Hauptbestandteile; und
zusätzlich zu den Hauptbestandteilen einen oberflächenaktiven Stoff, der kein nicht-ionischer oberflächenaktiver Stoff ist.

2. Desinfektionsmittel nach Anspruch 1, wobei das Metallion, das anti-mikrobielle Aktivität besitzt, ein dreiwertiges Eisenion (Fe³⁺), ein zweiwertiges Eisenion (Fe²⁺), ein Zinkion (Zn²⁺), ein Kupferion (Cu²⁺), ein Kobaltion (Co²⁺) ein Nickelion (Ni²⁺) oder ein Silberion (Ag⁺) ist.

3. Desinfektionsmittel nach Anspruch 2, wobei das Metallion, das anti-mikrobielle Aktivität besitzt, eine Konzentration von 50 bis 200 ppm hat, falls es sich um ein dreiwertiges Eisenion handelt, von 110 bis 400 ppm, falls es sich um ein zweiwertiges Eisenion handelt, von 7,5 bis 125 ppm, falls es sich um ein Zinkion handelt, von 15 bis 60 ppm, falls es sich um ein Kupferion handelt, von 180 bis 300 ppm, falls es sich um ein Kobaltion handelt, von 85 bis 175 ppm, falls es sich um ein Nickelion handelt, und von 1 bis 3 ppm, falls es sich um ein Silberion handelt.

4. Desinfektionsmittel nach einem der Ansprüche 1 bis 3, wobei das L-Cystein eine Konzentration von 100 bis 1000 ppm und die L-Ascorbinsäure eine Konzentration von 100 bis 500 ppm hat.

5. Desinfektionsmittel nach einem der Ansprüche 1 bis 4, wobei der oberflächen-aktive Stoff, der kein nicht-ionischer oberflächenaktiver Stoff ist, ausgewählt ist aus der Gruppe bestehend aus einem Alkylbenzolsulfonat, linearen Alkylbenzolsulfonat, Polyoxyethylen-Alkylether-Sulfat, höheren Alkoholsulfat, Natriumlaurylsulfat, Natriumlauroylsarkosinat, Stearyl-dimethyl-benzyl-ammoniumchlorid, Benzalkoniumchlorid, Benzethoniumchlorid, Alkyldiaminoethylglycin-hydrochlorid und Alkylpolyaminoethylglycin-hydrochlorid.

6. Desinfektionsmittel nach einem der Ansprüche 1 bis 5, wobei der oberflächen-aktive Stoff, der kein nicht-ionischer oberflächenaktiver Stoff ist, eine Konzentration von 20 bis 100 ppm hat.

7. Desinfektionsmittel nach einem der Ansprüche 1 bis 6, weiter umfassend einen oder mehrere Stoffe ausgewählt aus der Gruppe bestehend aus Sorbinsäure, Sorbat, Benzoesäure, Benzoat und para-Oxybenzoat.

8. Desinfektionsmittel nach Anspruch 7, wobei die Sorbinsäure, das Sorbat, die Benzoesäure, das Benzoat und das para-Oxybenzoat jeweils eine Konzentration von 50 bis 100 ppm haben.

9. Desinfektionsmittel nach einem der Ansprüche 1 bis 8, wobei das Desinfektionsmittel so eingestellt ist, dass es einen pH-Wert von 2,5 bis 4,0 hat.

10. Sterilisationsverfahren umfassend:
Inkontaktbringen des Desinfektionsmittels nach einem der Ansprüche 1 bis 9 mit einer Umgebung, einem Instrument, einem Pflanzenkörper oder einer organischen Substanz.

11. Desinfektionsmittel nach einem der Ansprüche 1 bis 9 zur Verwendung in der Begleitbehandlung von Infektionen in Menschen und Tieren.

12. Desinfektionsmittel nach einem der Ansprüche 1 bis 9 zur Verwendung in der Desinfizierung oder Sterilisation der Haut oder Mukosa des menschlichen oder tierischen Körpers.

## Revendications

1. Désinfectant comprenant :
un ion métallique ayant une activité antimicrobienne, de la L-cystéine et de l'acide L-ascorbique en tant que composants principaux ; et
en plus des composants principaux, un tensioactif autre qu'un tensioactif non ionique.

2. Désinfectant selon la revendication 1, dans lequel l'ion métallique ayant une activité antimicrobienne est un ion fer trivalent (Fe³⁺), un ion fer divalent (Fe²⁺), un ion zinc (Zn²⁺), un ion cuivre (Cu²⁺), un ion cobalt (Co²⁺), un ion nickel (Ni²⁺) ou un ion argent (Ag⁺).

3. Désinfectant selon la revendication 2, dans lequel l'ion métallique ayant une activité antimicrobienne a une concentration de 50 à 200 ppm dans le cas de l'ion fer trivalent, de 110 à 400 ppm dans le cas de l'ion fer divalent, de 7,5 à 125 ppm dans le cas de l'ion zinc, de 15 à 60 ppm dans le cas de l'ion cuivre, de 180 à 300 ppm dans le cas de l'ion cobalt, de 85 à 175 ppm dans le cas de l'ion nickel, et de 1 à 3 ppm dans le cas de l'ion argent.

4. Désinfectant selon l'une quelconque des revendications 1 à 3, dans lequel la L-cystéine a une concentration de 100 à 1000 ppm et l'acide L-ascorbique a une concentration de 100 à 500 ppm.

5. Désinfectant selon l'une quelconque des revendications 1 à 4, dans lequel le tensioactif autre qu'un tensioactif non ionique est un ou plusieurs tensioactifs choisis dans le groupe constitué par un sulfonate d'alkylbenzène, un sulfonate d'alkylbenzène linéaire, un sulfate d'éther alkylique de polyoxyéthylène, un sulfate d'alcool élevé, le laurylsulfate de sodium, le lauroylsarcosinate de sodium, le chlorure de stéaryldiméthylbenzylammonium, le chlorure de benzalkonium, le chlorure de benzéthonium, un chlorhydrate d'alkyldiaminoéthylglycine et un chlorhydrate d'alkylpolyaminoéthylglycine.

6. Désinfectant selon l'une quelconque des revendications 1 à 5, dans lequel le tensioactif autre qu'un tensioactif non ionique a une concentration de 20 à 100 ppm.

7. Désinfectant selon l'une quelconque des revendications 1 à 6, comprenant en outre un ou plusieurs composés choisis dans le groupe constitué par l'acide sorbique, un sorbate, l'acide benzoïque, un benzoate et un para-oxybenzoate.

8. Désinfectant selon la revendication 7, dans lequel chacun de l'acide sorbique, sorbate, acide benzoïque, benzoate et para-oxybenzoate a une concentration de 50 à 100 ppm.

9. Désinfectant selon l'une quelconque des revendications 1 à 8, le désinfectant étant ajusté à un pH de 2,5 à 4,0.

10. Méthode de stérilisation comprenant :
la mise en contact du désinfectant selon l'une quelconque des revendications 1 à 9 avec un environnement, un instrument, un corps végétal, ou une substance organique.

11. Désinfectant selon l'une quelconque des revendications 1 à 9, pour une utilisation dans le traitement auxiliaire d'infection chez l'homme et les animaux.

12. Désinfectant selon l'une quelconque des revendications 1 à 9, pour une utilisation dans la désinfection ou la stérilisation de la peau ou d'une muqueuse d'un corps humain ou animal.
